# EUROPEAN PATENT APPLICATION

(11) **EP 4 378 941 A1**
(43) Date of publication of application: **05.06.2024**
(21) Application number: 22879569.6
(22) Date of filing: 20.10.2022
(51) Int. Cl.: C07D 417/14, A61P 31/04, A61K 31/433

(54) **SALT FORM OF COMPOUND HAVING SUBSTITUTED SULFONYLUREA RING AND CRYSTAL FORM THEREOF**

(30) Priority: 12.10.2022 CN 202211246849
(71) Applicant: Suzhou Erye Pharmaceutical Co., Ltd., Suzhou, Jiangsu 215131 (CN)
(72) Inventor: HUANG, Zhigang, Shanghai 200131 (CN); LUO, Wei, Shanghai 200131 (CN); HU, Lihong, Shanghai 200131 (CN); CHEN, Shuhui, Shanghai 200131 (CN); LIU, Zhi, Suzhou, Jiangsu 215131 (CN); LU, Wenjuan, Suzhou, Jiangsu 215131 (CN)
(74) Representative: Herzog IP Patentanwalts GmbH
(86) International application number: PCT/CN2022/126339
(87) International publication number: WO 2024/077650

(57) **Abstract**

The present disclosure provides a salt form and a crystal form of a sulfonylurea ring substituted compound and a specific crystal form A thereof, and also provides an application thereof in preparation of medicaments for treating bacterial infection-related diseases. The crystal form A of the compound is easy to obtain and has good physical stability and chemical stability, with high industrial application value and economic value. The compound of the present disclosure has good water solubility, suitable for intravenous administration, and excellent in pharmacokinetic properties; has good safety; has good efficacy, and will be used for treatment of bacterial infection-related diseases.

## Description

### TECHNICAL FIELD

The present disclosure relates to a salt form and a crystal form thereof of a sulfonylurea ring substituted compound, and particularly discloses a compounds shown in formula (I), a crystal form thereof, a preparation method therefor, a pharmaceutically acceptable salts thereof, and an applications thereof in the preparation of medicaments for treating related diseases.

### BACKGROUND OF THE INVENTION

β-lactam antibiotics are important antibiotics. However, with drug-resistance genes and antibiotic hydrolases such as extended spectrum serine β-lactamases (ESBLs) and serine carbapenemases (such as KPCs) constantly emerging, the efficacy of existing antibiotics can no longer meet the requirements. Especially for serious and even multi-drug resistance mediated by metallo β-lactamases (MBLs), traditional antibiotics such as penicillins, cephalosporins, carbapenems, etc. are no longer able to cope with infections caused by these resistant bacteria. As a countermeasure, monocyclic β-lactam ring antibiotics represented by aztreonam are naturally stable to metallo β-lactamase (MBLs) and are a highly advantageous chemical series.

The marketed drug aztreonam has disadvantages of poor permeability, strong efflux effect, narrow antibacterial spectrum, instability to extended spectrum serine β-lactamases (ESBLs) and the like. To overcome these clinical problems, the strategy of enhancing permeability through siderophores has been introduced. For example, Basilea (WO 2007065288) and Naeja Pharmaceuticals (WO 2002022613) reported corresponding series of molecules; recently, Novartis (WO 2015148379) reported the study of overcoming drug resistance by modifying the substituents of the aztreonam molecule, and the formula of the compounds are as shown below, wherein the group Het is a heteroaromatic ring or a heterocycle substituted with 1-2 heteroatoms.

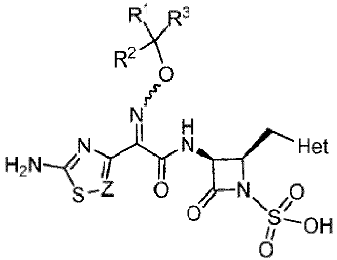

In view of the severe situation of drug resistance, it is an urgent task to develop a new generation of monocyclic β-lactam antibiotics to solve the current drug resistance problem. The sulfonylurea ring substituted monocyclic β-lactam molecule in the present disclosure has the corresponding potential.

The patent PCT/CN2020/130568 recorded a sulfonylurea ring substituted monocyclic β-lactam compound, which are mainly used for the treatment of bacterial infection-related diseases. Its structure is as shown in formula (B-I):

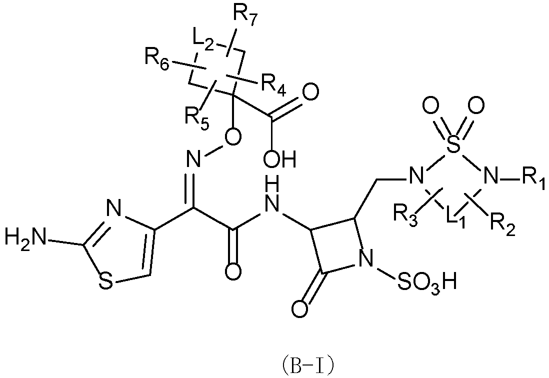

### SUMMARY OF THE INVENTION

The present disclosure provides a crystal form A of the compound of formula (I), and the X-ray powder diffraction pattern thereof has characteristic diffraction peaks at the following 2θ angles: 15.05±0.20°, 19.11±0.20°, and 21.15±0.20°.

In some embodiments of the present disclosure, the X-ray powder diffraction pattern of the crystal form A described above has characteristic diffraction peaks at the following 2θ angles: 15.05±0.20°, 19.11±0.20°, 20.41±0.20°, 21.15±0.20°, 22.62±0.20°, 23.48±0.20°, 27.32±0.20°, and 29.25±0.20°.

In some embodiments of the present disclosure, the X-ray powder diffraction pattern of the crystal form A described above has characteristic diffraction peaks at the following 2θ angles: 9.10±0.20°, 11.29±0.20°, 12.76±0.20°, 15.05±0.20°, 16.39±0.20°, 19.11±0.20°, 20.41±0.20°, 21.15±0.20°, 22.62±0.20°, and 23.68±0.20°.

In some embodiments of the present disclosure, the X-ray powder diffraction pattern of the crystal form A described above has characteristic diffraction peaks at the following 2θ angles: 6.90±0.20°, 9.10±0.20°, 11.29±0.20°, 11.83±0.20°, 12.76±0.20°, 13.73±0.20°, 14.57±0.20°, 15.05±0.20°, 16.10±0.20°, 16.39±0.20°, 17.81±0.20°, 18.25±0.20°, 19.11±0.20°, 20.41±0.20°, and 21.15±0.20°.

In some embodiments of the present disclosure, the X-ray powder diffraction pattern of the crystal form A described above has characteristic diffraction peaks at the following 2θ angles: 6.90±0.20°, 9.10±0.20°, 11.29±0.20°, 11.83±0.20°, 12.76±0.20°, 13.73±0.20°, 15.05±0.20°, 16.39±0.20°, 17.81±0.20°, 19.11±0.20°, 20.41±0.20°, 21.15±0.20°, 21.64±0.20°, 22.62±0.20°, and 23.48±0.20°.

In some embodiments of the present disclosure, the X-ray powder diffraction pattern of the crystal form A described above has characteristic diffraction peaks at the following 2θ angles: 6.90±0.20°, 9.10±0.20°, 11.29±0.20°, 11.83±0.20°, 12.76±0.20°, 13.73±0.20°, 15.05±0.20°, 16.39±0.20°, 17.81±0.20°, 19.11±0.20°, 20.41±0.20°, 21.15±0.20°, 21.64±0.20°, 22.62±0.20°, 23.48±0.20°, 25.73±0.20°, 26.43±0.20°, 27.32±0.20°, 28.42±0.20°, and 29.25±0.20°.

In some embodiments of the present disclosure, the X-ray powder diffraction pattern of the crystal form A described above has characteristic diffraction peaks at the following 2θ angles: 15.05±0.20°, 19.11±0.20°, 21.15±0.20°, and/or 6.90±0.20°, and/or 9.10±0.20°, and/or 11.29±0.20°, and/or 11.83±0.20°, and/or 12.76±0.20°, and/or 13.73±0.20°, and/or 14.57±0.20°, and/or 16.10±0.20°, and/or 16.39±0.20°, and/or 17.81±0.20°, and/or 18.25±0.20°, and/or 20.41±0.20°, and/or 20.79±0.20°, and/or 21.64±0.20°, and/or 22.62±0.20°, and/or 23.48±0.20°, and/or 23.68±0.20°, and/or 24.00±0.20°, and/or 24.81±0.20°, and/or 25.73±0.20°, and/or 26.43±0.20°, and/or 26.66±0.20°, and/or 27.32±0.20°, and/or 27.64±0.20°, and/or 28.42±0.20°, and/or 29.25±0.20°, and/or 29.92±0.20°, and/or 30.36±0.20°, and/or 30.73±0.20°, and/or 31.51±0.20°, and/or 31.83±0.20°, and/or 32.05±0.20°, and/or 32.51±0.20°, and/or 32.84±0.20°, and/or 33.13±0.20°, and/or 33.84±0.20°, and/or 34.35±0.20°, and/or 34.90±0.20°, and/or 35.50±0.20°, and/or 36.04±0.20°, and/or 36.47±0.20°, and/or 37.10±0.20°, and/or 37.57±0.20°, and/or 37.88±0.20°, and/or 38.80±0.20°.

In some embodiments of the present disclosure, the X-ray powder diffraction pattern of the crystal form A described above has characteristic diffraction peaks at the following 2θ angles: 6.904°, 9.095°, 11.285°, 11.833°, 12.757°, 13.732°, 14.573°, 15.050°, 16.100°, 16.389°, 17.807°, 18.251°, 19.113°, 20.413°, 20.790°, 21.147°, 21.641°, 22.615°, 23.484°, 23.676°, 24.002°, 24.812°, 25.728°, 26.433°, 26.655°, 27.323°, 27.636°, 28.420°, 29.248°, 29.922°, 30.358°, 30.733°, 31.507°, 31.830°, 32.049°, 32.509°, 32.843°, 33.130°, 33.837°, 34.346°, 34.895°, 35.500°, 36.037°, 36.467°, 37.098°, 37.573°, 37.875°, and 38.797°.

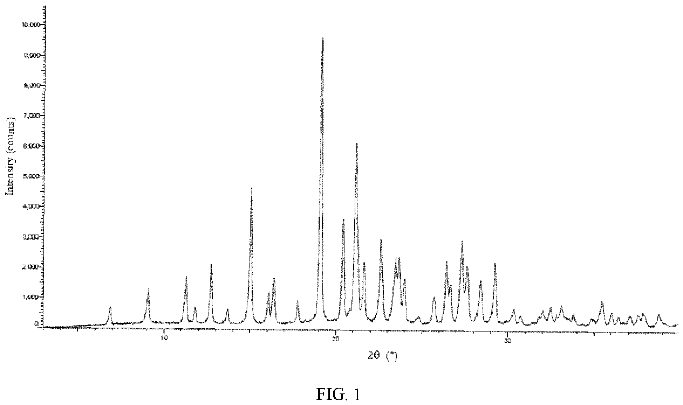

In some embodiments of the present disclosure, the XRPD pattern of the crystal form A described above is basically as shown in FIG. 1.

In some embodiments of the present disclosure, see Table 1 for the analytical data of the X-ray powder diffraction pattern of the crystal form A described above.

**Table 1 Analytic data of the X-ray powder diffraction pattern of the crystal form A of the compound of formula (I)**

| **No.** | **2θ angle(°)** | **D-spacing (Å)** | **Net intensity** | **Intensity** | **Relative intensity** |
|---|---|---|---|---|---|
| 1 | 6.904 | 12.79240 | 379.083 | 501.579 | 0.06 |
| 2 | 9.095 | 9.71589 | 791.659 | 961.096 | 0.12 |
| 3 | 11.285 | 7.83459 | 1062.760 | 1253.110 | 0.16 |
| 4 | 11.833 | 7.47281 | 349.972 | 542.781 | 0.05 |
| 5 | 12.757 | 6.93349 | 1300.330 | 1491.760 | 0.19 |
| 6 | 13.732 | 6.44340 | 364.829 | 547.393 | 0.06 |
| 7 | 14.573 | 6.07360 | 56.582 | 246.023 | 0.01 |
| 8 | 15.050 | 5.88197 | 3023.410 | 3222.600 | 0.45 |
| 9 | 16.100 | 5.50069 | 696.994 | 901.849 | 0.10 |
| 10 | 16.389 | 5.40428 | 1009.180 | 1211.780 | 0.15 |
| 11 | 17.807 | 4.97713 | 511.336 | 748.907 | 0.08 |
| 12 | 18.251 | 4.85694 | 43.230 | 300.544 | 0.01 |
| 13 | 19.113 | 4.63970 | 6685.510 | 6978.030 | 1.00 |
| 14 | 20.413 | 4.34713 | 2353.140 | 2674.880 | 0.35 |
| 15 | 20.790 | 4.26916 | 290.038 | 615.257 | 0.04 |
| 16 | 21.147 | 4.19780 | 4065.760 | 4391.700 | 0.61 |
| 17 | 21.641 | 4.10312 | 1354.850 | 1677.640 | 0.20 |
| 18 | 22.615 | 3.92861 | 1978.170 | 2292.990 | 0.30 |
| 19 | 23.484 | 3.78517 | 1531.180 | 1831.550 | 0.23 |
| 20 | 23.676 | 3.75490 | 1468.580 | 1763.760 | 0.22 |
| 21 | 24.002 | 3.70460 | 1030.740 | 1315.430 | 0.15 |
| 22 | 24.812 | 3.58557 | 154.093 | 403.730 | 0.02 |
| 23 | 25.728 | 3.45985 | 603.966 | 860.868 | 0.09 |
| 24 | 26.433 | 3.36913 | 1469.410 | 1748.670 | 0.22 |
| 25 | 26.655 | 3.34161 | 888.799 | 1173.080 | 0.13 |
| 26 | 27.323 | 3.26146 | 1935.580 | 2229.130 | 0.29 |
| 27 | 27.636 | 3.22516 | 1342.450 | 1637.320 | 0.20 |
| 28 | 28.420 | 3.13796 | 1050.320 | 1340.050 | 0.16 |
| 29 | 29.248 | 3.05102 | 1526.200 | 1797.390 | 0.23 |
| 30 | 29.922 | 2.98383 | 76.617 | 326.849 | 0.01 |
| 31 | 30.358 | 2.94189 | 368.856 | 607.042 | 0.06 |
| 32 | 30.733 | 2.90686 | 219.512 | 444.382 | 0.03 |
| 33 | 31.507 | 2.83721 | 59.538 | 279.903 | 0.01 |
| 34 | 31.830 | 2.80916 | 198.932 | 425.732 | 0.03 |
| 35 | 32.049 | 2.79044 | 355.647 | 585.642 | 0.05 |
| 36 | 32.509 | 2.75201 | 424.627 | 658.254 | 0.06 |
| 37 | 32.843 | 2.72482 | 261.023 | 494.689 | 0.04 |
| 38 | 33.130 | 2.70181 | 469.399 | 701.331 | 0.07 |
| 39 | 33.837 | 2.64694 | 287.940 | 508.712 | 0.04 |
| 40 | 34.346 | 2.60892 | 41.629 | 249.459 | 0.01 |
| 41 | 34.895 | 2.56908 | 135.360 | 349.547 | 0.02 |
| 42 | 35.500 | 2.52669 | 589.156 | 816.636 | 0.09 |
| 43 | 36.037 | 2.49026 | 320.094 | 553.358 | 0.05 |
| 44 | 36.467 | 2.46189 | 175.090 | 408.889 | 0.03 |
| 45 | 37.098 | 2.42142 | 261.939 | 489.944 | 0.04 |
| 46 | 37.573 | 2.39193 | 283.100 | 501.597 | 0.04 |
| 47 | 37.875 | 2.37356 | 336.537 | 546.685 | 0.05 |
| 48 | 38.797 | 2.31924 | 309.914 | 508.468 | 0.05 |

In some embodiments of the present disclosure, the thermogravimetric analysis curve of the crystal form A described above has a weight loss of 6.657% at 140.000°C±3.000°C and a weight loss of 12.576% at 205.000°C±3.000°C.

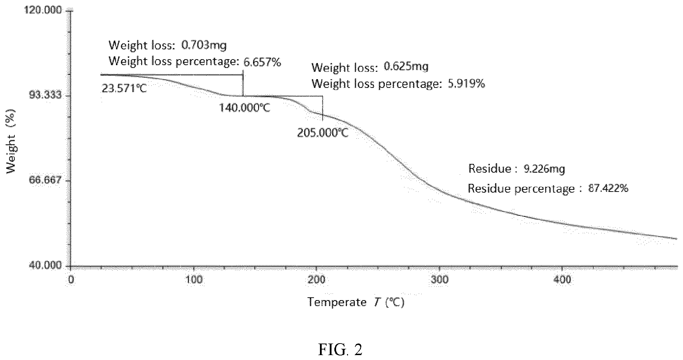

In some embodiments of the present disclosure, the thermogravimetric analysis curve of the crystal form A described above is basically as shown in FIG. 2.

In some embodiments of the present disclosure, the differential scanning calorimetric curve of the crystal form A described above has an endothermic peak at 100.28°C±3.00°C, an endothermic peak at 127.61°C±3.00°C, an exothermic peak at 200.70°C ±3.00°C, and an endothermic peak at 266.36°C±3.00°C.

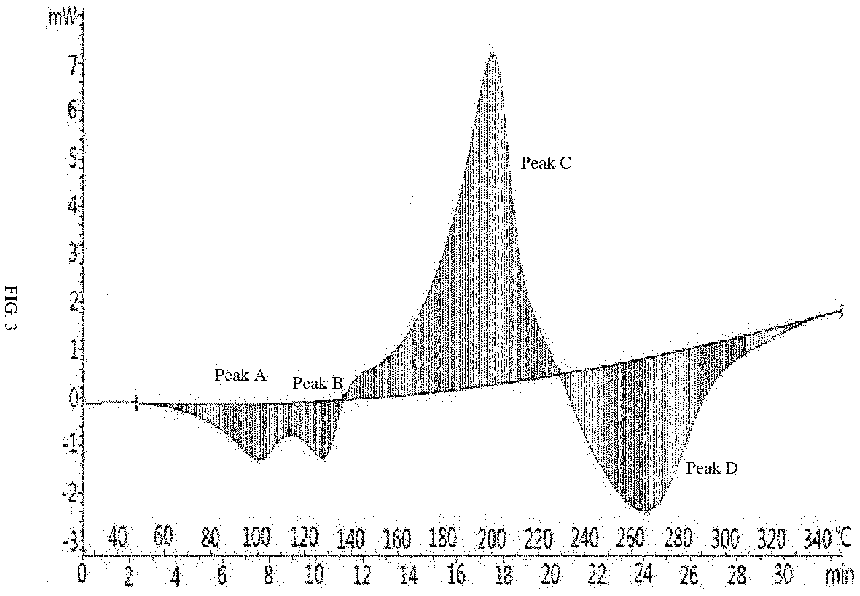

In some embodiments of the present disclosure, the differential scanning calorimetric curve of the crystal form A described above is basically as shown in FIG. 3

The present disclosure also provides an application of the compound of formula (I) or the crystal form thereof in the preparation of medicaments for treating bacterial infection-related diseases.

### Technical Effect

The crystal form A of the compound of formula (I) of the present disclosure is easy to obtain and has good physical stability and chemical stability, with high industrial application value and economic value. The present disclosure discloses a novel sulfonylurea ring substituted monocyclic β-lactam compound, which is stable to.serine β-lactamases and metallo β-lactamases. It has a broad antibacterial spectrum against gram-negative bacteria, and especially has prominent antibacterial activity against various carbapenem-resistant enterobacteriaceae superbacteria. The compound disclosed by the present disclosure has good water solubility, is suitable for intravenous administration, and has excellent pharmacokinetic properties; has high safety; has good efficacy, and will be used for treatment of bacterial infection-related diseases.

### Definitions and Explanations

The present disclose uses the following abbreviations: aq refers to water; BOC refers to tert-butoxycarbonyl, which is an amine protecting group; TEMPO refers to 2,2,6,6-tetramethylpiperidine nitroxide; TCCA refers to Trichloroisocyanuric acid; HCl refers to hydrochloric acid; Pd/C refers to palladium carbon; TFA refers to trifluoroacetic acid; DCM refers to dichloromethane; MeCN refers to acetonitrile; (*n*-Bu)₄NHSO₄ refers to tetrabutyl ammonium bisulfate; IPA refers to isopropanol; *eq* refers to equivalent; CBz refers to benzyloxycarbonyl, which is an amine protection group.

### BRIEF DESCRIPTION OF THE DRAWINGS

FIG. 1 is the XRPD pattern of the crystal form A of the compound of formula (I);
FIG. 2 is the TGA pattern of the crystal form A of the compound of formula (I);
FIG. 3 is the DSC pattern of the crystal form A of the compound of formula (I) (detail information of Peak A: integral -189.19mJ, normalized -82.26 Jg^-1, onset 74.62°C, peak 100.28°C, endset 120.89°C, left limit 47.75°C, right limit 113.15°C; detail information of Peak B: integral -113.80mJ, normalized -49.48 Jg^-1, onset 105.72°C, peak 127.61°C, endset 136.22°C, left limit 113.15°C, right limit 136.62°C; detail information of Peak C: integral 1374.45mJ, normalized 597.59 Jg^-1, onset 170.56 °C, peak 200.70 °C, endset 215.91 °C, left limit 136.62°C, right limit 228.98°C; detail information of Peak D: integral -888.43mJ, normalized -386.27 Jg^-1, onset 229.07 °C, peak 266.36 °C, endset 299.00 °C, left limit 228.98°C, right limit 350.12°C);
FIG. 4 is the simulated XRPD pattern (wavelength 1.54056; 14.585, 10366; h,k,l=4,0,-1) of the single crystal of the compound of formula (I).

### DETAILED DESCRIPTION OF THE PREFERRED EMBODIMENTS

The present disclosure is described in detail in the following embodiments, but it does not mean any adverse limitation to the invention. The compounds of the present disclosure can be prepared by a variety of synthesis methods familiar to those skilled in the art, including the specific embodiments listed below, the embodiments formed by combining them with other chemical synthesis methods of the compounds, and the equal replacement methods familiar to those skilled in the art. The preferred embodiments include but are not limited to the embodiments of the present disclosure. It will be apparent to those skilled in the art to make various changes and improvements to the specific embodiments of the present disclosure without departing from the spirit and scope of the present disclosure.

### Embodiment 1: Compound of Formula (I)

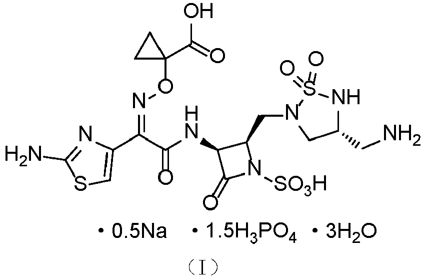

### Preparation of Intermediate A:

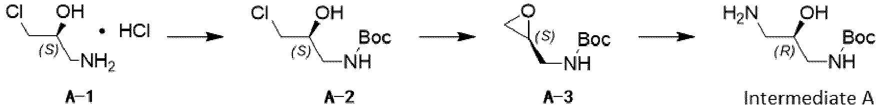

### Step 1: Preparation of Compound A-2

Compound A-1 (1.00 kg, 6.85 mol, 1 *eq)* was dissolved in methanol (4 L), Boc anhydride (1.63 kg, 7.46 mol, 1.71 L, 1.09 *eq)* was slowly added dropwise at 20°C. Potassium carbonate (1.09 kg, 7.88 mol, 1.15 *eq)* was dissolved in water (2 L), slowly added dropwise to the reaction system and stirred at 20~30°C for 1 hours. The reaction solution was added with ethyl acetate (8 L) for extraction twice, and the organic phases were combined and concentrated under reduced pressure. The residue was added with ethyl acetate (15 L), washed with water (10 L) once, dried over anhydrous sodium sulfate, filtered, and concentrated under vacuum to obtain Compound A-2. ¹H NMR (400 MHz, CDCl₃) δ = 5.01 (br s, 1H), 3.93 (br s, 1H), 3.65-3.50 (m, 2H), 3.44 (br d, *J=* 14.0 Hz, 1H), 3.33-3.19 (m, 1H), 1.46 (d, *J=* 1.9 Hz, 9H).

### Step 2: Preparation of Compound A-3

Compound A-2 (1.15 kg, 5.48 mol, 1 *eq)* was dissolved in ethanol (1.5 L), and the temperature was lowered to 0~5°C. Sodium hydroxide (338.50 g, 6.03 mol, 1.1 *eq)* was dissolved in ethanol (4 L) and slowly added to the reaction system, after addition the temperature was slowly lifted to 25°C, and it was stirred at 25°C for 1 hour. The reaction solution was added with 1N HCl to adjust pH=7, and concentrated under reduced pressure. The residue was added with ethyl acetate (5 L × 2) and washed with salt water (1 L) once, the organic phases were combined, dried over anhydrous sodium sulfate, filtered and concentrated under reduced pressure. The residue was dissolved in a mixed solvent of petroleum ether: ethyl acetate =3:1 (5 L), added with 100-200 mesh silica gel (1.50 kg) at 15°C, stirred for 0.5 hour and filtered, the filter cake was washed with a mixed solvent (5 L) of petroleum ether/ethyl acetate =1/3 three times, and the filtrate was combined and concentrated under vacuum to obtain Compound A-3. ¹H NMR (400 MHz, CDCl₃) δ = 4.76 (br s, 1H), 3.62-3.50 (m, 1H), 3.28-3.19 (m, 1H), 3.11 (br s, 1H), 2.80 (t, *J* = 4.3 Hz, 1H), 2.61 (dd, *J* = 2.6, 4.7 Hz, 1H), 1.46 (s, 9H).

### Step 3: Preparation of Intermediate A

Compound A-3 was dissolved in ethanol (5 L), ammonia water (4.55 kg, 32.46 mol, 5 L, 25% purity, 5.62 *eq)* was added at 0~5°C, after addition the temperature was lifted to 20°C, and it was stirred for 16 hour. The reaction solution was distilled under reduced pressure, and the residue was added with solid sodium chlorid and stirred at 25°C for 0.5 hour. Ethyl acetate (2 L) was added for extraction once, the aqueous phase was extracted with ethyl acetate (500 mL), and the organic phases were combined, dried over anhydrous sodium sulfate, filtered and concentrated under reduced pressure. The residue was triturated with petroleum ether/tert butyl methyl ether =1/1 at 20°C for 1 hour and filtered, and the filter cake was dried under vacuum to obtain Intermediate A. ¹H NMR (400 MHz, CDCl₃) δ = 5.46-5.08 (m, 1H), 3.79-3.54 (m, 1H), 3.32-3.19 (m, 1H), 3.13-2.99 (m, 1H), 2.79 (dd, *J* = 4.0, 12.7 Hz, 1H), 1.43 (s, 9H).

### Preparation of Intermediate B

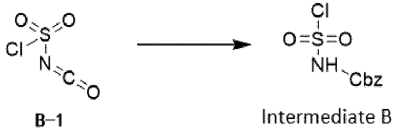

Compound B-1 (84.00 g, 593.50 mmol, 1 *eq)* was dissolved in dichloromethane (590 mL), and slowly added dropwise to a dichloromethane (590 mL) solution of benzyl alcohol (66.11g, 661.30mmol, 1.03 *eq)* at 0-10°C under nitrogen atmosphere, and the reaction solution was reacted in ice bath for 0.5h. The reaction solution was concentrated under vacuum to obtain a crude product, and the crude product was triturated with petroleum ether (600 mL) at 25°C for 15 minutes and filtered to obtain Intermediate B. ¹H NMR (400 MHz, CDCl₃) δ = 7.51 - 7.36 (m, 5H), 5.33 (s, 2H).

### Preparation of Intermediate C

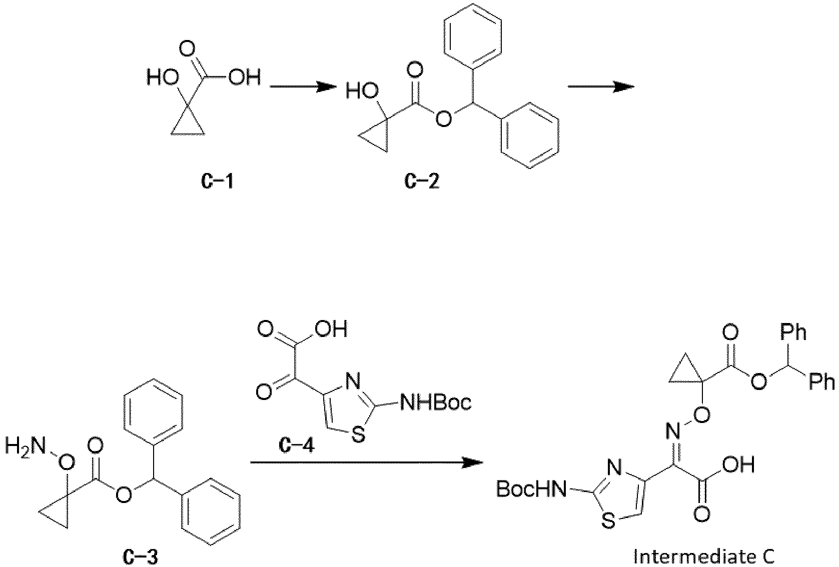

### Step 1: Preparation of Compound C-2

1.8-diazabicyclo[5.4.0]undecyl-7-ene (536.8 g, 3.53 mol, 531.5 mL, 1.2 *eq)* was added to a toluene (3.5 L) solution of Compound C-1 (300.0 g, 2.94 mol, 1 *eq)* and bromodiphenylmethane (835.2 g, 3.38 mol, 1.15 *eq)* at 20-30°C, and then the temperature was lifted to 75~80°C for reaction for 5 hours. The reaction solution was cooled to room temperature, added with water (1 L) and stirred for 0.5h, the organic phase was separated, washed with water(1 L × 2), concentrated to about 500mL under reduced pressure, added with n-heptane (2 L), heated to 75~80°C to obtain a clear solution, cooled to room temperature, stirred for 1 hour in ice water and filtered, the filter cake was washed with toluene/*n*-heptane (600 mL, 1/5) and n-heptane (300 mL), the solid was dried by oil pump to obtain Compound C-2. ¹H NMR (400 MHz, CDCl₃) δ = 7.40-7.29 (m, 11H), 6.95 (s, 1H), 1.46-1.39 (m, 2H), 1.27-1.19 (m, 2H).

### Step 2: Preparation of Compound C-3

Compound C-2 (150.0 g, 559.1 mmol, 1 *eq)* and diphenylphosphorylhydroxylamine (156.5 g, 670.9 mmol, 1.2 *eq)* in tetrahydrofuran (1.5 L) solution was added with sodium tert butoxide in batches at 0-10°C, the temperature range was controlled to be 0~10°C, and the reaction was carried out under nitrogen protection at 0~10°C. The reaction solution was added with 5% sodium chloride solution (1 L) and stirred for 0.5 h, the organic phase was separated, the aqueous phase was extracted with ethyl acetate (500 mL × 2), and the organic phases were combined, washed with 5% sodium chloride solution (1 L × 2), dried over anhydrous sodium sulfate and filtered to obtain 2.5 L tetrahydrofuran/ethyl acetate solution of Compound C-3, which was directly used for the next reaction.

### Step 3: Preparation of Intermediate C

Compound C-4 (106.6 g, 391.3 mmol, 0.7 *eq)* was added to 2.5 L tetrahydrofuran/ethyl acetate solution of compound C-3 (158.4 g, 559.1 mmol, 1 *eq),* and the reaction was carried out at 20~30°C for 18 h. The reaction solution was concentrated under reduced pressure to obtain a residue, then added with ethyl acetate (400 mL), heated to 65~70°C and stirred for 0.5 hour, *n*-heptane (1.6 L) was added, the temperature was lowered to room temperature, it was stirred in ice bath for 0.5 h and filtered, and the filter cake was washed with ethyl acetate/ *n*-heptane (400 mL, 1/3) and dried by oil pump under reduced pressure to obtain a residue. The residue was added to acetonitrile (580 mL) and acetone (1.45 L), stirred at 55~60°C for 3 hour, concentrated under reduced pressure at 40°C to remove acetone, added with acetonitrile (400 mL) and concentrated under reduced pressure twice repeatedly, then added with acetonitrile (360 mL) and heated to 60°C, then water (580 mL) was added dropwise, it was cooled to room temperature, continuously stirred for 1 hour and filtered, the filter cake was sequentially washed with acetonitrile / water (300 mL, 1/1) and ice acetonitrile (100 mL), the solid was dried by oil pump under reduced pressure to obtained a residue. ¹H NMR (400 MHz, DMSO-*d*₆) δ = 11.83 (br s, 1H), 7.49 (s, 1H), 7.45 (dd, *J* = 1.8, 7.6 Hz, 4H), 7.30 - 7.21 (m, 6H), 6.88 (s, 1H), 1.51 (br d, *J* = 3.0 Hz, 2H), 1.48 (s, 9H), 1.45 - 1.40 (m, 2H).

### Preparation of the Compound of Formula (I):

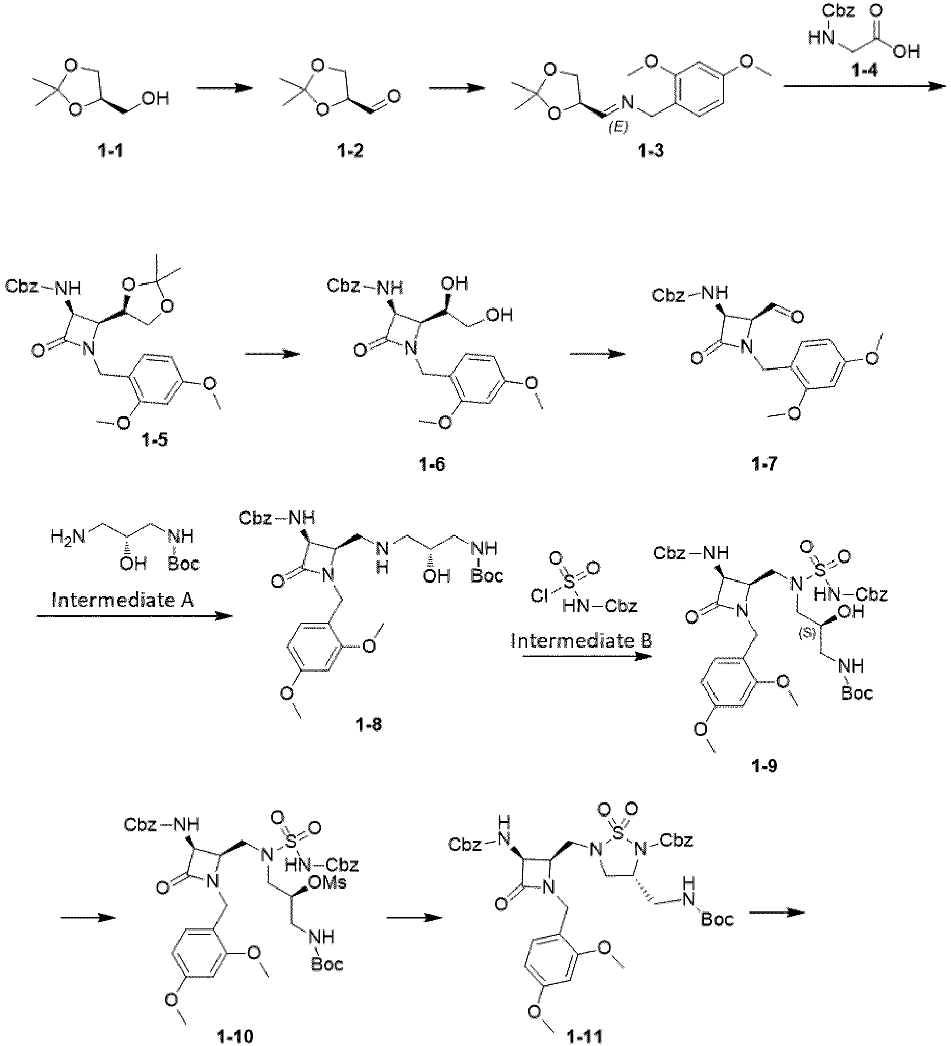

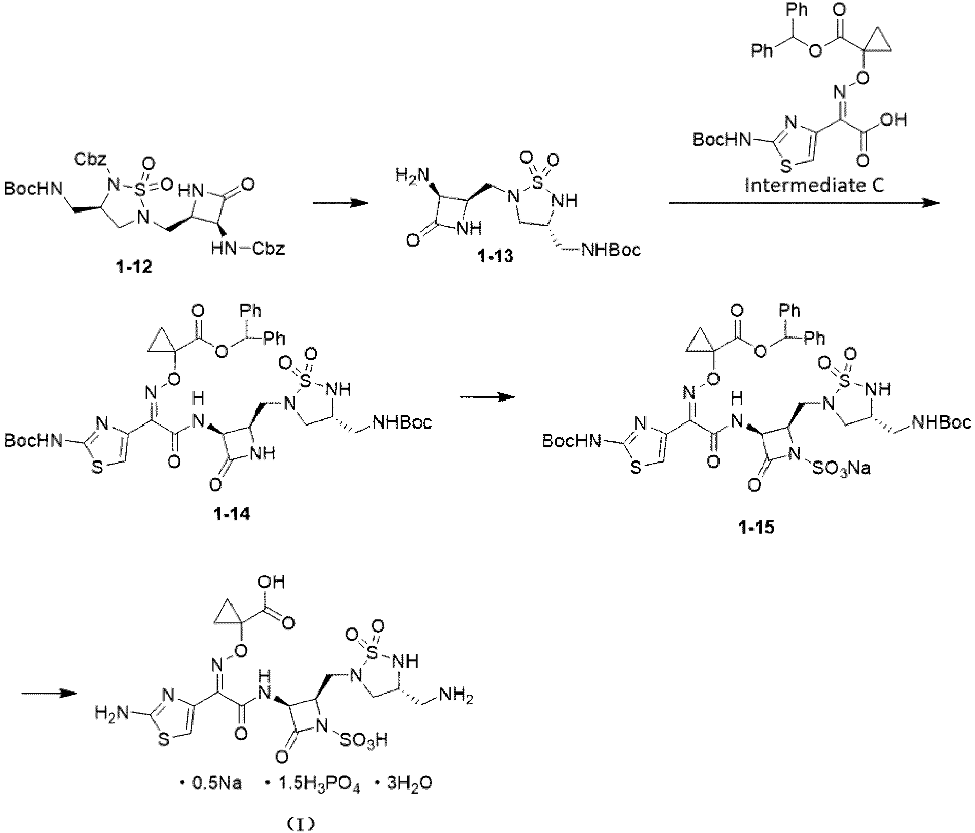

### Step 1: Preparation of Compound 1-2

Compound 1-1 (1.5 kg, 11.35 mol, 1 *eq)* was dissolved in DCM (15 L), added with TEMPO (4.46 g, 28.38 mmol, 0.0025 *eq)* and sodium acetate (1.40 kg, 17.03 mol, 1.5 *eq)* at once at 10~20°C under nitrogen protection, and then added with TCCA (1.06 kg,4.54 mol, 0.4 *eq)* in batches for 1 hour at 0-10°C, the reaction solution was heated to 10-20°C, continuously stirred for 1 hour and filtered, and the filtrate was a dichloromethane solution of Compound 1-2, which was directly used for the next reaction.

### Step 2: Preparation of Compound 1-3

1 equivalent of sodium carbonate aqueous solution was added to the dichloromethane solution of Compound 1-2 described above, then 2,4-dimethoxybenzylamine (1.52 kg, 9.10 mol, 1.37 L, 1 *eq)* was added, the mixture was stirred at 10~15°C for 0.5 hour. The reaction was stopped, the liquid was separated, the organic phase was washed with saturated salt water (5.0 L), dried over anhydrous sodium sulfate and filtered, and the filtrate was a dichloromethane solution of Compound 1-3, which was directly used for the next reaction.

### Step 3: Preparation of Compound 1-5

Compound 1-4 (2.47 kg, 11.82 mol, 1.3 *eq)* was dissolved in dichloromethane(12.8 L), the temperature was lowered to -40°C, triethylamine (3.59 kg, 35.46 mol, 4.94 L, 3.9 *eq)* and isobutyl chloroformate (4.84 kg, 35.46 mol, 4.66 L, 3.9 *eq)* were added, it was stirred at -30- -20°C for 30 minute, the dichloromethane solution of Compound 1-3 described above was added slowly at -20~ - 30°C, then the temperature was lifted to -20 ~10°C, continuously stirred for 6 hours. The reaction was stopped, sequentially washed with 3M HCl (6 L), a saturated sodium bicarbonate aqueous solution (6 L) and salt water (6 L), dried over anhydrous sodium sulfate and filtered, the filtrate was concentrated under reduced pressure to obtain a crude product, and the crude product was suspended in methanol, stirred in suspension at -30 - -20°C for 2 hours and filtered to obtain Compound 1-5 (880.0g, three-step total yield 18.5%).

### Step 4: Preparation of Compound 1-6

Compound 1-5 (131.0 g, 278.42 mmol, 1 *eq)* was added to tetrahydrofuran (1.2 L) and water (300 mL) at 20-30°C, p-toluenesulfonic acid monohydrate (37.07 g, 194.89 mmol, 0.7 *eq)* was added, it was stirred at 65°C for 4 hours. It was cooled to 20°C, adjusted to pH 7 with a saturated sodium bicarbonate solution and concentrated under reduced pressure at 40°C to remove tetrahydrofuran, the remaining mixture was added with water (1.2 L), stirred at 20~30°C for 1 hour and filtered, and the filter cake was washed to obtain Compound 1-6.

### Step 5: Preparation of Compound 1-7

Compound 1-6 (119.85 g, 278.43 mmol, 1 *eq)* was added to ethyl acetate (1.2 L) and water (400 mL) at 20-30°C, sodium periodate (65.51 g, 306.27 mmol, 1.1 *eq)* was added, and the mixture was stirred at 50°C for 2 hours. The temperature was lowered to 20~25°C, the organic phase and the aqueous phase were separated, the aqueous phase was extracted with ethyl acetate (300 mL × 2), the organic phases were combined, washed with 5% sodium chloride solution (300 mL) and concentrated under reduced pressure, and the crude product wad stirred with ethyl acetate (350 mL) at 20~30°C for 5 hours and filtered to obtain Compound 1-7. ¹H NMR (400 MHz, CDCl₃) δ = 9.35 (d, *J* = 3.3 Hz, 1H), 8.22 (d, *J* = 8.4 Hz, 1H), 7.42-.27 (m, 6H), 7.19 (d, *J* = 8.3 Hz, 1H), 6.57 (d, *J* = 2.1 Hz, 1H), 6.51 (dd, *J* = 2.3, 8.3 Hz, 1H), 5.01 (s, 2H), 4.97-4.90 (m, 1H), 4.47-4.24 (m, 2H), 4.08 (dd, *J* = 3.3, 5.8 Hz, 1H), 3.76 (s, 3H), 3.73 (s, 3H).

### Step 6: Preparation of Compound 1-8

Compound 1-7 (78.00 g, 195.80 mmol, 1 *eq)* and 4A molecular sieves (150.00 g) was added to anhydrous dichloromethane (780 mL) at 0°C, the dichloromethane (200 mL) solution of Intermediate A (55.87 g, 293.67 mmol, 1.5 *eq)* was added dropwise, it was stirred under nitrogen protection for 1 hour and filtered, the filtrate was added dropwise to an acetic acid (200 mL) solution of sodium triacetoxyborohydride (82.99 g, 391.56 mmol, 2 *eq),* it was stirred under nitrogen protection at 15~25°C for 16 hours. It was concentrated under reduced pressure at 35°C, then diluted with water (1 L), extracted with ethyl acetate (800 mL × 2), dried over anhydrous sodium sulfate and filtered, the filtrate was added with petroleum ether (1.5 L) and stirred at 15~25°C for 1 hour, and a white solid was precipitated and filtered to obtain Compound 1-8. ¹H NMR (400 MHz, DMSO) δ = 9.57-8.96 (m, 1H), 8.88-8.41 (m, 1H), 8.17 (br d, *J* = 9.3 Hz, 1H), 7.44-7.28 (m, 5H), 7.19 (d, *J* = 8.3 Hz, 1H), 6.90 (br s, 1H), 6.58 (d, *J* = 2.3 Hz, 1H), 6.50 (dd, *J* = 2.3, 8.3 Hz, 1H), 5.61 (br s, 1H), 5.15-5.01 (m, 2H), 4.99 (dd, *J* = 5.1, 9.4 Hz, 1H), 4.45-4.20 (m, 2H), 3.98 (br s, 1H), 3.80 (s, 3H), 3.76 (s, 3H), 3.15 (br s, 1H), 3.06 (br s, 1H), 2.99-2.90 (m, 2H), 2.84 (br d, *J* = 10.9 Hz, 1H), 2.64 (br t, *J* = 10.2 Hz, 1H), 1.38 (s, 9H).

### Step 7: Preparation of Compound 1-9

Compound 1-8 (70.00 g, 122.24 mmol, 1 *eq)* and triethylamine (49.48 g, 488.96 mmol, 68.06 mL, 4 *eq)* were added to anhydrous tetrahydrofuran (700 mL), the anhydrous tetrahydrofuran (300 mL) solution of Intermediate B (46.69 g, 187.03 mmol, 1.53 *eq)* was added dropwise at 0°C, and the mixture was stirred at 0~5°C for 1 hour. The anhydrous tetrahydrofuran (50 mL) solution of Intermediate B (6.10 g, 24.45 mmol, 0.2 *eq)* was added dropwise to the mixture,and stirred at 15~25°C for 1 hour to obtain a tetrahydrofuran solution of Compound 1-9, which was directly used for the next reaction.

### Step 8: Preparation of Compound 1-11

Triethylamine (25.09 g, 247.96 mmol, 34.51 mL, 2 *eq)* was added to the anhydrous tetrahydrofuran (1.08 L) solution of Compound 1-9 (97.43 g, 123.98 mmol, 1 *eq),* methanesulfonyl chloride (17.31 g, 151.11 mmol, 11.70 mL, 1.22 *eq)* was added dropwise at 0-10°C, it was stirred for 3 hour, methanesulfonyl chloride (16.16 g, 141.07 mmol, 10.92 mL, 1.14 *eq)* was added dropwise to the mixture, it was stirred at 0~10°C for 3 hours, triethylamine (25.09 g, 247.96 mmol, 34.51 mL, 2 *eq)* was added, it was stirred at 80°C for 1 hour, the temperature was lowered to 15-20°C, the mixture was poured into ice water (1.5 L) and extracted by ethyl acetate (1 L × 2), and the combined organic phase was washed with salt water (1 L), dried over anhydrous sodium sulfate, concentrated under reduced pressure, then added with ethyl acetate (500 mL), stirred at 15~25°C for 16 hours and filtered to obtain Compound 1-11. LCMS(ESI) m/z: 768.4(M+1).

### Step 9: Preparation of Compound 1-12

Compound 1-11 (20.00 g, 26.05 mmol, 1 *eq)* was dissolved in acetonitrile (200 mL) and water (100 mL), the temperature was lifted to the internal temperature 65~70°C in a 100°C oil bath, potassium persulfate (31.68 g, 117.21 mmol, 4.5 *eq)* and potassium dihydrogen phosphate (18.15 g, 104.19 mmol, 4 *eq)* were added at once, the reaction was carried out by refluxing for 1.5 hours. Then the temperature was lowered to room temperature, filtered, added with saturated sodium bicarbonate (200 mL) and extracted with ethyl acetate (200 mL × 2). The organic layer was washed with salt water (200 mL), dried over anhydrous sodium sulfate, filtered and concentrated under reduced pressure to obtain a residue. The residue was purified by flash silica gel column chromatography (petroleum ether/ethyl acetate =3/1 to 1/3, 10% dichloromethane) to obtain Compound 1-12. ¹H NMR (400 MHz, CDCl₃) δ = 7.44-7.20 (m, 11H), 6.02-5.50 (m, 1H), 5.31-5.16 (m, 2H), 5.11-4.90 (m, 4H), 4.02-3.97 (m, 1H), 3.97-3.86 (m, 1H), 3.70-3.44 (m, 2H), 3.43-3.09 (m, 3H), 2.97-2.81 (m, 1H), 1.89-1.57 (m, 1H), 1.36 (s, 9H).

### Step 10: Preparation of Compound 1-13

Compound 1-12 (300.0 g) and 3.0 L methanol was put into a 5L glass reaction flask and started to stir, the temperature was lifted to 40°C, it was stirred until the starting materials was dissolved. Pd/C (15.0g, 10% purity) was added under nitrogen protection, triethylsilyl hydrogen 564.8g beginned to added dropwise, the nitrogen flow environment was kept and the stirring continued. Pd/C (15.0g, 10% purity) was added under nitrogen protection, triethylsilyl hydrogen was kept to be added dropwise, nitrogen protection was kept after addition, the heating was turned off, and the stirring continued for 20 minutes. The reaction solution was suction filtered with diatomite, the filter cake was washed with methanol (500 ml × 2) twice, and the filtrate was concentrated at 45°C. The residue was added with 5.0 L tert-butyl methyl ether, stirred and triturated at 15~30°C for 18 hours and suction filtered under nitrogen protection, the filter cake was washed with 1.0 L tert-butyl methyl ether, suction filtered and sucked dry by oil pump rotary evaporator (temperature: 50°C, time: 2 hours) to obtain Compound 1-13.

### Step 11: Preparation of Compound 1-14

Compound 1-13 (20.50 g, 58.67 mmol, 1 *eq)* and Intermediate C (28.39 g, 52.80 mmol, 0.9 *eq)* were dissolved in *N,N*-dimethylformamide (200 mL), *N*,*N*-diisopropylethylamine (22.75 g, 176.01 mmol, 30.66 mL, 3 *eq)* and 1-hydroxybenzotriazole (10.31 g, 76.27 mmol, 1.3 *eq)* was added, then 1-(3-dimethylaminopropyl) -3-ethylcarbodiimide hydrochloride (14.62 g, 76.27 mmol, 1.3 *eq)* was added, and the reaction solution was reacted for 16 hours at 15~25°C under nitrogen protection. The reaction solution was poured into 1.5 L ice water, stirred for 10 minutes and then filtered, the filter cake was washed with water (300 mL × 2), the filtrate was extracted by ethyl acetate (500 mL × 2). The filter cake was dissolved into the organic phase, sequentially washed with dilute hydrochloric acid (0.5 N, 100 mL × 2), saturated sodium bicarbonate (100 mL × 2) and salt water (200 mL), dried over anhydrous sodium sulfate, filtered and concentrated under reduced pressure to obtain a residue. The residue was purified by flash silica gel column chromatography (petroleum ether/ethyl acetate =2/1 to 1/2, 10% dichloromethane) to obtain Compound 1-14.

### Step 12: Preparation of Compound 1-15

Compound 1-14 (10.00 g, 11.51 mmol, 1 *eq)* was dissolved in *N,N*- dimethylformamide (50 mL), sulfur trioxide *N,N*-dimethylformamide complex (13.63 g, 46.03 mmol, 47% purity, 4 *eq)* was added at 0~5°C, and it was stirred at 0~5°C for 3 hours. The mixture was poured into a mixed solvent of water (80 mL) and ethyl acetate (150 mL), the liquid was separated, the aqueous phase was extracted with ethyl acetate (100 mL × 3). The organic layer is washed with salt water (150 mL × 3), dried over anhydrous sodium sulfate, filtered and concentrated under reduced pressure to obtain a residue. The residue was purified by silicone column chromatography (petroleum ether/ethyl acetate=2/1 to 0/1 +10% dichloromethane) to obtain compound 1-15. LCMS (ESI) m/z: 949.1 (M+1).

### Step 13: Preparation of the Compound of Formula (I)

Compound 1-15 (6.20 g, 6.53 mmol, 1 *eq)* and anisole (7.06 g, 65.33 mmol, 7.10 mL, 10 *eq)* were dissolved in dichloromethane (60 mL), phosphoric acid (50.40 g, 437.14 mmol, 85% purity, 66.91 *eq)* was added at 0~5°C, and it was stirred for 1.5 hours at 0~5°C. The lower phosphoric acid phase of the mixture was added dropwise into tetrahydrofuran (300 mL), it was stirred at 0~5°C for 1 hour and filtered under nitrogen protection, the filter cake was washed with tetrahydrofuran (100 mL × 2) to obtain the crude product compound of formula (I), which was an amorphous powder detected by XRPD. The crude product was dissolved in water (4 mL) and filtered, the filtrate was added dropwise into tetrahydrofuran (80 mL), the mixture was stirred at 25~30°C for 4 hours and filtered under nitrogen protection, the filter cake was washed with tetrahydrofuran (50 mL × 2) and dried to obtain the compound of formula (I). The compound of formula (I) was crystal form A detected by XRPD. The natrium ion content was 1.59% and the phosphate radical content was 19.4% detected by ICPMS, and the water content was 7.5% shown by Karl Fischer moisture titrating test, determining that the compound of formula (I) was a salt form containing 0.5 natrium and 1.5 phosphoric acid and a crystal form A containing three crystalline water. LCMS (ESI) m/z: 583.1 (M+1). ¹H NMR (400 MHz, DMSO-*d*₆ + D₂O) δ = 6.80 (s, 1H), 5.23 (d, *J* = 5.7 Hz, 1H), 4.17 (q, *J* = 5.7 Hz, 1H), 3.84 - 3.76 (m, 2H), 3.67 (br t, *J* = 8.9 Hz, 1H), 3.42 (br dd, *J* = 5.1, 14.4 Hz, 1H), 3.16 (br dd, *J* = 6.2, 14.2 Hz, 1H), 3.04 (br dd, *J* = 5.4, 9.9 Hz, 1H), 2.98 - 2.89 (m, 1H), 2.88 - 2.79 (m, 1H), 1.29 (br d, *J* = 9.4 Hz, 4H).

### Embodiment 2: Crystal Form A of the Compound of Formula (I)

### Method one:

The crude product of the compound of formula (I) (0.50 g, 685.33 µmol) was dissolved in water (0.5 mL), filtered to remove insoluble impurities, added dropwise into tetrahydrofuran (10 mL) at 25~30°C and stirred at 25~30°C for 4 hours, and after 4 hours it was dispersed evenly, filtered under nitrogen protection and dried to obtain the crystal form A of the compound of formula (I).

### Method two:

The compound of formula (I) (100 mg, 137.07 µmol) was dissolved in water (0.1 mL), tetrahydrofuran (2mL) was added dropwise to the solution to have solid precipitated, it was stirred for 2 hours to start solidification and dispersed gradually, and after 4 hours it was dispersed evenly, filtered under nitrogen protection and dried to obtain the crystal form A of the compound of formula (I).

### Method three:

The compound of formula (I) (20.00 mg, 27.4 µmol) was added separately with a mixed solvent of water and tetrahydrofuran (1/15 = 0.5 mL), (1/20 = 0.5 mL), (1/25=0.5 mL), and stirred at 25-30°C, the solid was dispersed evenly after about 5 hours, filtered under nitrogen protection and dried to obtain the crystal form A of the compound of formula (I).

### Method four:

The compound of formula (I) (500 mg, 685.33 µmol) was dissolved in water (1.0 mL), 85% phosphoric acid (67.16 mg, 685.33 mmol) was added, tetrahydrofuran (20.0 mL) was added dropwise to the solution, and it was stirred at 15~25°C for 12 hours, filtered under nitrogen protection and dried to obtain the crystal form A of the compound of formula (I).

### Method five:

The compound of formula (I) (500 mg, 685.33 µmol) was dissolved in water (1.0 mL), 85% phosphoric acid (67.16 mg, 685.33 mmol) was added, and isopropanol (6.0 mL) was added dropwise to the solution, and it was stirred at 15~25°C for 12 hours, filtered under nitrogen protection and dried to obtain the crystal form A of the compound of formula (I).

### Embodiment 3: Configuration Confirmation of the Compound of Formula (I)

16mg of a sample of crystal form A of formula (I) was taken and dissolved in 900µL methanol/ethanol/water (1:1:1) at room temperature, and the sample solution was placed in a 4 mL semi-sealed sample vial and slowly evaporated at room temperature. A colorless needle crystal was obtained on the second day.

The crystal was detected to be colorless needle-like (0.30 × 0.10 × 0.10 mm³), belonging to monoclinic C2 space group. Cell parameters: a = 25.3958(7) Å, b = 8.0781(2) Å, c = 14.8880(2) Å, *α* = 90°, *β* = 90.224(2)°, *γ* = 90°, V = 3054.25(12) Å³, Z = 2. The calculated density Dc = 1.728g/cm³, the number of electrons in the unit cell F(000) = 1648.0, the linear absorption coefficient of the unit cell *µ*(Cu Kα) = 3.918mm⁻¹, and the temperature of the diffraction experiment T = 149.99(11) K.

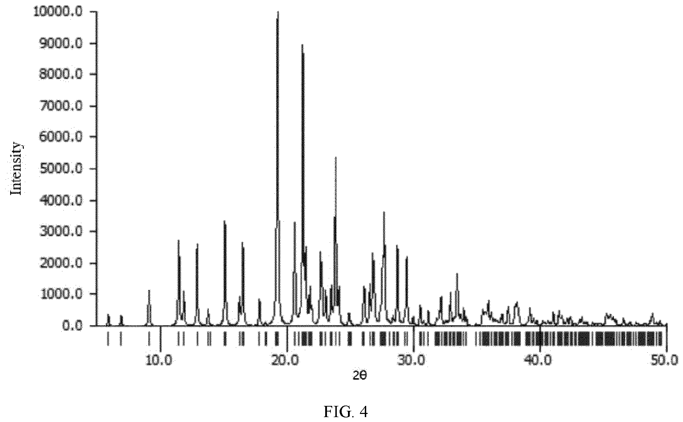

For the simulated XRPD of the above needle crystal see FIG. 4, and for structural data and parameters see Table 2~7.

**Table 2 Crystal structural data and determination parameters of the compound of formula (I)**

| Empirical formula | C₁₆H₂₂N₈O₁₀S₃•0.5Na•1.5H₃PO₄•3H₂O |
|---|---|
| Formula weight | 582.58/795.11 |
| Temperature | T = 149.99(11) K |
| Crystal system | monoclinic |
| Space group | C2 |
| Unit cell dimensions | a/Å 25.3958(7) |
| | b/Å 8.0781(2) |
| | b/Å 14.8880(2) |
| Volume /Å | 3054.25(12) |
| Z | 2 |
| Calculated density calc g/cm³ | 1.728 |
| Absorption coefficient µ/mm⁻¹ | 3.918 |
| F(000) | 1648.0 |
| Crystal size | 0.30 × 0.10 × 0.10 |
| Angle range for data collection | -30 ≤ h ≤ 30, -9 ≤ k ≤ 9, -14 ≤ 1 ≤ 17 |
| Reflection collection | 26495/5372 [Rᵢₙₜ = 0.0576] |
| Goodness-of -fit on F2 | 1.053 |
| Final R indices [I>2sigma (I)] | R₁ = 0.0803, wR₂ = 0.2276 |
| R indices (All Data) | R₁ = 0.0814, wR₂ = 0.2295 |
| Largest diffraction peak and hole/e Å⁻³ | 0.96/-0.71 |

**Table 3 Atomic coordinates (× 10⁴) and equivalent isotropic displacement parameters (Å2 × 10³) of the crystal of the compound of formula (I)**

| **Atom** | **x** | **y** | **z** | **U(eq)** |
|---|---|---|---|---|
| S(3) | 7748.3(7) | -249(2) | 2047.0(11) | 36.2(5) |
| S(1) | 5742.5(10) | 10722(3) | 2513.3(19) | 57.8(6) |
| P(2) | 10000 | 2823(5) | 5000 | 52.7(8) |
| S(2) | 6166.6(12) | 955(4) | -290.0(16) | 64.8(7) |
| P(1) | 4388.8(11) | 2517(3) | 3269(2) | 60.1(7) |
| O(9) | 7659(3) | -1637(8) | 1479(4) | 49.0(15) |
| O(4) | 6159(2) | 4599(8) | 2011(3) | 41.5(12) |
| Na(1) | 5000 | 3180(11) | 0 | 81.1(19) |
| O(3) | 7105(3) | 4727(8) | 3389(4) | 48.6(14) |
| O(10) | 7458(3) | -273(9) | 2868(3) | 51.8(15) |
| N(4) | 6891(3) | 5676(9) | 1392(4) | 40.0(14) |
| O(2) | 8300(2) | 4788(10) | 4643(4) | 54.9(16) |
| O(5) | 5923(3) | 5021(11) | -23(4) | 61.6(19) |
| O(1) | 8029(3) | 6144(9) | 3431(4) | 52.9(16) |
| N(3) | 6873(3) | 6258(10) | 3548(4) | 44.6(16) |
| N(5) | 6438(3) | 2629(10) | 204(4) | 45.2(16) |
| N(6) | 7645(3) | 1471(8) | 1470(4) | 37.0(14) |
| O(8) | 5656(4) | 978(12) | -9(7) | 86(3) |
| N(2) | 6305(3) | 9184(10) | 3633(5) | 45.8(16) |
| O(14) | 4737(4) | 1839(15) | 4013(8) | 91(3) |
| O(6) | 6466(4) | -450(11) | 60(6) | 87(3) |
| O(11) | 3837(4) | 2883(14) | 3475(8) | 88(3) |
| C(9) | 6532(3) | 5546(10) | 2044(5) | 34.9(15) |
| C(10) | 6303(4) | 4293(11) | 197(5) | 43.3(18) |
| C(13) | 7080(3) | 1918(10) | 1414(5) | 37.9(16) |
| O(12) | 4410(3) | 1091(11) | 2553(6) | 72(2) |
| N(7) | 8375(4) | 99(11) | 2143(7) | 65(2) |
| C(5) | 7445(3) | 4309(11) | 4098(5) | 42.8(18) |
| C(15) | 8510(4) | 1848(12) | 1994(6) | 45.6(19) |
| O(13) | 4633(4) | 4013(12) | 2872(7) | 78(2) |
| C(11) | 6856(3) | 4780(11) | 566(4) | 37.9(16) |
| O(16) | 9912(4) | 3828(11) | 4167(6) | 79(2) |
| O(19) | 5175(5) | 8008(17) | 8894(8) | 102(4) |
| N(8) | 9364(4) | 2305(12) | 2772(6) | 63(2) |
| O(17) | 5096(3) | 4494(15) | 1313(7) | 84(3) |
| C(12) | 6978(3) | 2884(11) | 544(5) | 39.1(17) |
| C(8) | 7984(3) | 5094(12) | 4111(4) | 50(2) |
| O(15) | 9496(4) | 1778(13) | 5015(7) | 83(2) |
| N(1) | 5973(4) | 11620(13) | 4223(8) | 68(2) |
| C(7) | 7418(4) | 2515(12) | 4338(6) | 52(2) |
| C(4) | 6598(3) | 6680(11) | 2840(5) | 39.6(16) |
| C(1) | 6027(4) | 10561(14) | 3572(7) | 54(2) |
| C(3) | 6304(3) | 8205(11) | 2859(6) | 41.0(17) |
| C(6) | 7210(4) | 3751(12) | 4978(5) | 50(2) |
| O(18) | 4440(3) | 4627(12) | 6373(6) | 73(2) |
| O(7) | 6256(4) | 1088(16) | -1247(6) | 89(3) |
| C(2) | 6016(4) | 8848(12) | 2189(6) | 46.8(19) |
| C(16) | 8809(4) | 2631(13) | 2785(6) | 53(2) |
| C(14) | 7994(4) | 2731(11) | 1833(6) | 45.7(18) |

**Table 4 Bond length (Å) of the crystal of the compound of formula (I)**

| **Atom** | **Atom** | **Bond length / Å** | **Atom** | **Atom** | **Bond length / Å** |
|---|---|---|---|---|---|
| S(3) | O(9) | 1.422(6) | N(4) | C(9) | 1.338(10) |
| S(3) | O(10) | 1.430(6) | N(4) | C(11) | 1.429(10) |
| S(3) | N(6) | 1.653(6) | O(2) | C(8) | 1.1509(15) |
| S(3) | N(7) | 1.622(10) | O(5) | C(10) | 1.176(11) |
| S(1) | C(1) | 1.737(11) | O(1) | C(8) | 1.326(9) |
| S(1) | C(2) | 1.734(9) | N(3) | C(4) | 1.308(11) |
| P(2) | O(16) | 1.498(9) | N(5) | C(10) | 1.387(12) |
| P(2) | O(16)¹ | 1.498(9) | N(5) | C(12) | 1.474(11) |
| P(2) | O(15)¹ | 1.534(10) | N(6) | C(13) | 1.483(11) |
| P(2) | O(15) | 1.534(10) | N(6) | C(14) | 1.452(11) |
| S(2) | N(5) | 1.685(8) | N(2) | O(1) | 1.320(14) |
| S(2) | O(8) | 1.365(11) | N(2) | C(3) | 1.397(11) |
| S(2) | O(6) | 1.461(10) | C(9) | C(4) | 1.506(10) |
| S(2) | O(7) | 1.447(9) | C(10) | C(11) | 1.556(11) |
| P(1) | O(14) | 1.517(11) | C(13) | C(12) | 1.534(10) |
| P(1) | O(11) | 1.466(10) | N(7) | C(15) | 1.471(13) |
| P(1) | O(12) | 1.571(10) | C(5) | C(8) | 1.511(12) |
| P(1) | O(13) | 1.483(10) | C(5) | C(7) | 1.495(13) |
| O(4) | C(9) | 1.219(10) | C(5) | C(6) | 1.510(11) |
| Na(1) | O(5) | 2.777(9) | C(15) | C(16) | 1.536(11) |
| Na(1) | O(5)² | 2.777(9) | C(15) | C(14) | 1.510(13) |
| Na(1) | O(8) | 2.437(12) | C(11) | C(12) | 1.563(12) |
| Na(1) | O(8)² | 2.437(12) | N(8) | C(16) | 1.435(14) |
| Na(1) | 0(17) | 2.237(12) | N(1) | C(1) | 1.300(14) |
| Na(1) | O(17)² | 2.237(12) | C(7) | C(6) | 1.479(13) |
| O(3) | N(3) | 1.391(10) | C(4) | C(3) | 1.441(12) |
| O(3) | C(5) | 1.402(10) | C(3) | C(2) | 1.340(13) |

| | | | | | |
|---|---|---|---|---|---|
| ¹2-X,+Y,1-Z; ²1-X,+Y,-Z | | | | | |

**Table 5 Bond angle of the crystal of the compound of formula (I)**

| **Atom** | **Atom** | **Atom** | **Bond angle /°** | **Atom** | **Atom** | **Atom** | **Bond angle /°** |
|---|---|---|---|---|---|---|---|
| O(9) | S(3) | O(10) | 114.6(4) | C(12) | N(5) | S(2) | 129.8(6) |
| O(9) | S(3) | N(6) | 109.2(3) | C(13) | N(6) | S(3) | 112.6(5) |
| O(9) | S(3) | N(7) | 110.1(5) | C(14) | N(6) | S(3) | 107.5(5) |
| O(10) | S(3) | N(6) | 112.0(4) | C(14) | N(6) | C(13) | 116.1(7) |
| O(10) | S(3) | N(7) | 115.9(5) | S(2) | O(8) | Na(1) | 131.5(7) |
| N(7) | S(3) | N(6) | 93.0(4) | C(1) | N(2) | C(3) | 114.9(8) |
| C(2) | S(1) | C(1) | 91.3(5) | O(4) | C(9) | N(4) | 123.5(7) |
| O(16)¹ | P(2) | 0(16) | 114.4(7) | O(4) | C(9) | C(4) | 119.8(7) |
| O(16) | P(2) | O(15)¹ | 114.1(5) | N(4) | C(9) | C(4) | 116.7(7) |
| O(16) | P(2) | 0(15) | 100.8(6) | O(5) | C(10) | N(5) | 133.5(9) |
| O(16)¹ | P(2) | O(15)¹ | 100.8(6) | O(5) | C(10) | C(11) | 135.3(9) |
| O(16)¹ | P(2) | 0(15) | 114.1(5) | N(5) | C(10) | C(11) | 91.2(6) |
| O(15)¹ | P(2) | 0(15) | 113.2(9) | N(6) | C(13) | C(12) | 109.4(6) |
| O(8) | S(2) | N(5) | 104.0(5) | C(15) | N(7) | S(3) | 112.5(7) |
| O(8) | S(2) | O(6) | 113.3(7) | O(3) | C(5) | C(8) | 117.7(7) |
| O(8) | S(2) | O(7) | 117.0(7) | O(3) | C(5) | C(7) | 112.7(7) |
| O(6) | S(2) | N(5) | 104.9(5) | O(3) | C(5) | C(6) | 118.7(8) |
| O(7) | S(2) | N(5) | 107.7(5) | C(7) | C(5) | C(8) | 116.5(8) |
| O(7) | S(2) | O(6) | 109.0(7) | C(7) | C(5) | C(6) | 59.0(6) |
| O(14) | P(1) | 0(12) | 102.1(6) | C(6) | C(5) | C(8) | 118.4(7) |
| O(11) | P(1) | 0(14) | 118.4(7) | N(7) | C(15) | C(16) | 113.2(8) |
| O(11) | P(1) | O(12) | 109.0(6) | N(7) | C(15) | C(14) | 106.0(7) |
| O(11) | P(1) | 0(13) | 108.6(6) | C(14) | C(15) | C(16) | 110.6(8) |
| O(13) | P(1) | 0(14) | 110.0(6) | N(4) | C(11) | C(10) | 119.0(6) |
| O(13) | P(1) | 0(12) | 108.2(6) | N(4) | C(11) | C(12) | 120.2(6) |
| O(5) | Na(1) | O(5)² | 115.2(5) | C(10) | C(11) | C(12) | 85.6(6) |
| O(8)² | Na(1) | O(5)² | 79.3(3) | N(5) | C(12) | C(13) | 111.9(7) |
| O(8) | Na(1) | O(5) | 79.3(3) | N(5) | C(12) | C(11) | 87.7(6) |
| O(8) | Na(1) | O(5)² | 165.5(4) | C(13) | C(12) | C(11) | 120.9(6) |
| O(8)² | Na(1) | O(5) | 165.5(4) | O(2) | C(8) | O(1) | 127.1(9) |
| O(8) | Na(1) | O(8)² | 86.2(6) | O(2) | C(8) | C(5) | 123.2(8) |
| O(17)² | Na(1) | O(5) | 79.9(3) | O(1) | C(8) | C(5) | 109.7(5) |
| O(17)² | Na(1) | O(5)² | 70.6(3) | C(6) | C(7) | C(5) | 61.0(6) |
| O(17) | Na(1) | O(5) | 70.6(3) | N(3) | C(4) | C(9) | 122.2(7) |
| O(17) | Na(1) | O(5)² | 79.9(3) | N(3) | C(4) | C(3) | 118.8(7) |
| O(17)² | Na(1) | O(8)² | 106.2(4) | C(3) | C(4) | C(9) | 118.6(7) |
| O(17)² | Na(1) | O(8) | 114.5(4) | N(2) | C(1) | S(1) | 110.2(7) |
| O(17) | Na(1) | O(8)² | 114.5(4) | N(1) | C(1) | S(1) | 125.6(9) |
| O(17) | Na(1) | O(8) | 106.2(4) | N(1) | C(1) | N(2) | 124.2(10) |
| O(17) | Na(1) | O(17)² | 123.3(7) | N(2) | C(3) | C(4) | 120.0(7) |
| N(3) | O(3) | C(5) | 110.2(6) | C(2) | C(3) | N(2) | 113.2(8) |
| C(9) | N(4) | C(11) | 123.0(7) | C(2) | C(3) | C(4) | 126.8(8) |
| C(10) | O(5) | Na(1) | 114.9(7) | C(7) | C(6) | C(5) | 60.0(6) |
| C(4) | N(3) | O(3) | 108.6(6) | C(3) | C(2) | S(1) | 110.4(7) |
| C(10) | N(5) | S(2) | 132.4(6) | N(8) | C(16) | C(15) | 113.4(8) |
| C(10) | N(5) | C(12) | 95.5(7) | N(6) | C(14) | C(15) | 104.8(7) |

| | | | | | | | |
|---|---|---|---|---|---|---|---|
| ¹2-X,+Y,1-Z; ²1-X,+Y,-Z | | | | | | | |

**Table 6 Hydrogen bond of the crystal of the compound of formula (I)**

| **Donor** | **Hydrogen** | **Receptor** | **d(D-H)/Å** | **d(H-A)/Å** | **d(D-A)/Å** | **D-H-A/°** |
|---|---|---|---|---|---|---|
| N(4) | H(4) | O(9)¹ | 0.86 | 2.08 | 2.921(10) | 164.1 |
| O(1) | H(1) | O(11)² | 0.82 | 1.84 | 2.487(13) | 134.4 |
| N(7) | H(7A) | O(11)³ | 0.89 | 2.14 | 2.914(15) | 145.1 |
| O(19) | H(19B) | O(17)⁴ | 0.85 | 2.28 | 2.937(18) | 134.0 |
| N(8) | H(8A) | O(19)⁵ | 0.89 | 1.92 | 2.804(15) | 174.6 |
| N(8) | H(8B) | O(13)³ | 0.89 | 1.87 | 2.749(14) | 167.4 |
| N(8) | H(8C) | O(16) | 0.89 | 1.90 | 2.782(12) | 170.0 |
| O(17) | H(17A) | O(13) | 0.99 | 1.65 | 2.635(14) | 171.3 |
| O(17) | H(17B) | O(4) | 0.93 | 2.04 | 2.890(10) | 151.1 |
| O(15) | H(15A) | O(18)³ | 0.82 | 1.86 | 2.670(14) | 167.7 |
| N(1) | H(1A) | O(2)⁶ | 0.86 | 2.19 | 2.903(14) | 140.4 |
| N(1) | H(1B) | O(18)⁷ | 0.86 | 1.97 | 2.790(15) | 159.0 |
| O(18) | H(18A) | O(4)⁴ | 0.85 | 2.00 | 2.851(10) | 174.9 |
| O(18) | H(18B) | O(13)⁴ | 0.85 | 1.82 | 2.653(13) | 164.6 |

| | | | | | | |
|---|---|---|---|---|---|---|
| ¹+X,1+Y,+Z; ²1/2+X,1/2+Y,+Z; ³1/2+X,-1/2+Y,+Z; ⁴1-X,+Y,1-Z; ⁵3/2-X,-1/2+Y,1-Z; ⁶3/2-X,1/2+Y1-Z; ⁷1-X,1+Y,1-Z | | | | | | |

**Table 7 Torsion angle of the crystal of the compound of formula (I)**

| **A** | **B** | **C** | **D** | **Angle / °** | **A** | **B** | **C** | **D** | **Angle / °** |
|---|---|---|---|---|---|---|---|---|---|
| S(3) | N(6) | C(13) | C(12) | -151.4(6) | N(2) | C(3) | C(2) | S(1) | 0.6(10) |
| S(3) | N(6) | C(14) | C(15) | 41.5(8) | O(6) | S(2) | N(5) | C(10) | -174.3(9) |
| S(3) | N(7) | C(15) | C(16) | 123.6(8) | O(6) | S(2) | N(5) | C(12) | 27.2(9) |
| S(3) | N(7) | C(15) | C(14) | 2.2(10) | O(6) | S(2) | O(8) | Na(1) | 163.0(7) |
| S(2) | N(5) | C(10) | O(5) | 14.4(16) | C(9) | N(4) | C(11) | C(10) | 28.7(11) |
| S(2) | N(5) | C(10) | C(11) | -164.1(7) | C(9) | N(4) | C(11) | C(12) | -74.0(10) |
| S(2) | N(5) | C(12) | C(13) | -72.7(9) | C(9) | C(4) | C(3) | N(2) | 172.0(8) |
| S(2) | N(5) | C(12) | C(11) | 164.7(6) | C(9) | C(4) | C(3) | C(2) | -8.5(13) |
| O(9) | S(3) | N(6) | C(13) | 82.1(6) | C(10) | N(5) | C(12) | C(13) | 123.1(7) |
| O(9) | S(3) | N(6) | C(14) | -148.8(6) | C(10) | N(5) | C(12) | C(11) | 0.5(6) |
| O(9) | S(3) | N(7) | C(15) | 131.3(7) | C(10) | C(11) | C(12) | N(5) | -0.4(5) |
| O(4) | C(9) | C(4) | N(3) | 91.2(10) | C(10) | C(11) | C(12) | C(13) | -114.8(8) |
| O(4) | C(9) | C(4) | C(3) | -82.1(10) | C(13) | N(6) | C(14) | C(15) | 168.6(6) |
| Na(1) | O(5) | C(10) | N(5) | 24.0(13) | N(7) | S(3) | N(6) | C(13) | -165.4(6) |
| Na(1) | O(5) | C(10) | C(11) | -158.2(8) | N(7) | S(3) | N(6) | C(14) | -36.4(7) |
| O(3) | N(3) | C(4) | C(9) | 4.4(11) | N(7) | C(15) | C(16) | N(8) | 83.1(11) |
| O(3) | N(3) | C(4) | C(3) | 177.7(7) | N(7) | C(15) | C(14) | N(6) | -26.9(10) |
| O(3) | C(5) | C(8) | O(2) | -177.0(9) | C(5) | O(3) | N(3) | C(4) | 174.1(7) |
| O(3) | C(5) | C(8) | O(1) | 2.8(11) | C(11) | N(4) | C(9) | O(4) | 3.6(12) |
| O(3) | C(5) | C(7) | C(6) | -110.9(8) | C(11) | N(4) | C(9) | C(4) | -174.8(7) |
| O(3) | C(5) | C(6) | C(7) | 100.5(9) | C(12) | N(5) | C(10) | O(5) | 177.9(10) |
| O(10) | S(3) | N(6) | C(13) | -45.9(6) | C(12) | N(5) | C(10) | C(11) | -0.5(6) |
| O(10) | S(3) | N(6) | C(14) | 83.2(6) | C(8) | C(5) | C(7) | C(6) | 108.7(8) |
| O(10) | S(3) | N(7) | C(15) | -96.7(8) | C(8) | C(5) | C(6) | C(7) | -105.4(9) |
| N(4) | C(9) | C(4) | N(3) | -90.3(10) | C(7) | C(5) | C(8) | O(2) | -38.6(12) |
| N(4) | C(9) | C(4) | C(3) | 96.4(9) | C(7) | C(5) | C(8) | O(1) | 141.2(7) |
| N(4) | C(11) | C(12) | N(5) | 120.7(7) | C(4) | C(3) | C(2) | S(1) | -178.9(7) |
| N(4) | C(11) | C(12) | C(13) | 6.4(11) | C(1) | S(1) | C(2) | C(3) | -0.6(8) |
| O(5) | C(10) | C(11) | N(4) | 59.8(13) | C(1) | N(2) | C(3) | C(4) | 179.3(8) |
| O(5) | C(10) | C(11) | C(12) | -177.9(10) | C(1) | N(2) | C(3) | C(2) | -0.3(12) |
| N(3) | O(3) | C(5) | C(8) | -79.5(9) | C(3) | N(2) | C(1) | S(1) | -0.2(10) |
| N(3) | O(3) | C(5) | C(7) | 140.6(8) | C(3) | N(2) | C(1) | N(1) | 178.9(9) |
| N(3) | O(3) | C(5) | C(6) | 74.7(9) | C(6) | C(5) | C(8) | O(2) | 28.7(14) |
| N(3) | C(4) | C(3) | N(2) | -1.6(12) | C(6) | C(5) | C(8) | O(1) | -151.5(8) |
| N(3) | C(4) | C(3) | C(2) | 177.9(9) | O(7) | S(2) | N(5) | C(10) | 69.7(10) |
| N(5) | S(2) | O(8) | Na(1) | 49.7(9) | O(7) | S(2) | N(5) | C(12) | -88.8(9) |
| N(5) | C(10) | C(11) | N(4) | -121.8(8) | O(7) | S(2) | O(8) | Na(1) | -68.9(10) |
| N(5) | C(10) | C(11) | C(12) | 0.5(6) | C(2) | S(1) | C(1) | N(2) | 0.5(7) |
| N(6) | S(3) | N(7) | C(15) | 19.6(8) | C(2) | S(1) | C(1) | N(1) | -178.6(9) |
| N(6) | C(13) | C(12) | N(5) | 150.3(7) | C(16) | C(15) | C(14) | N(6) | -150.0(7) |
| N(6) | C(13) | C(12) | C(11) | -108.6(8) | C(14) | N(6) | C(13) | C(12) | 84.1(8) |
| O(8) | S(2) | N(5) | C(10) | -55.1(10) | C(14) | C(15) | C(16) | N(8) | -158.1(9) |
| O(8) | S(2) | N(5) | C(12) | 146.4(8) | - | - | - | - | - |

### Embodiment 4: Preparation of Other Salt Forms of the Compound of Formula (I)

### (1) Preparation of Trifluoroacetate (TFA):

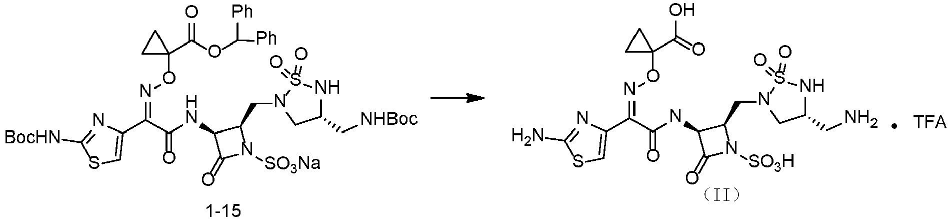

Intermediate 1-15 (300 mg, 308.95 µmοl, 1 *eq)* and anisole (66.82 mg, 617.91 µmοl, 67.16 µL, 2 *eq)* were dissolved in DCM (3 mL), and TFA (704.54 mg, 6.18 mmol, 457.49 µL, 20 *eq)* was added to the mixture at 0°C. Then the mixture was stirred at 0~5°C for 1 hour. Then the mixture was added with H₂O (140.04 mg, 7.77 mmol, 140.04 µL, 25.15 *eq)* at 0°C, and stirred at 25°C for 24 hours. The lower aqueous phase/TFA was added dropwise into the cold solution of MeCN (8 mL) at 0~5°C, and then the mixture was stirred at 0~5°C for 0.5 hour. The product was filtered under vacuum under nitrogen protection to obtain a crude product (110 mg, 168.67 µmοl, 54.59% yield, 89.33% purity), which was a grayish white solid. The solid was dissolved in 5 mL water, then adjusted to pH=7-8 with saturated sodium bicarbonate at 0°C, and then adjusted to pH=4-5 with (*n*-Bu)₄NHSO₄. It was purified by preparative HPLC (column: Waters Xbridge BEH C₁₈ 250*50mm*10µm; mobile phase: acetonitrile / water (10mM NH₄HCO₃), the percentage of acetonitrile is from 5% to 30%, 21 minutes), and then purified by preparative HPLC (column: Phenomenex Synergi Max-RP 250*50mm*10 µm; mobile phase: acetonitrile / water (0.1% TFA), the percentage of acetonitrile is from 0% to 25%, 21 minutes) to obtain the compound of formula (II), which was an amorphous powder detected by XRPD. LCMS (ESI) m/z: 583.1 (M+1). ¹H NMR (400 MHz, D₂O) δ = 6.80 (s, 1H), 5.23 (d, *J=* 5.7 Hz, 1H), 4.17 (q, *J* = 5.7 Hz, 1H), 3.84 - 3.76 (m, 2H), 3.67 (br t, *J* = 8.9 Hz, 1H), 3.42 (br dd, *J* = 5.1, 14.4 Hz, 1H), 3.16 (br dd, *J* = 6.2, 14.2 Hz, 1H), 3.04 (br dd, *J* = 5.4, 9.9 Hz, 1H), 2.98 - 2.89 (m, 1H), 2.88 - 2.79 (m, 1H), 1.29 (br d, *J* = 9.4 Hz, 4H).

### (2) Preparation of sulfate (H₂SO₄)

The reaction became complex, and no sulfate was obtained.

### (3) Preparation of hydrochloride (HCl)

The starting material was completely consumed, but no product was formed.

### (4) Preparation of acetate (AcOH)

The starting material was completely consumed, but no product was formed.

### (5) Preparation of p-toluenesulfonate (TsOH)

The starting material was completely consumed,but no product was formed.

Conclusion: Some of the other salt forms of the compound of formula (I) can not be obtained directly.

### Embodiment 5: Stability Test of the Crystal Form A of the Compound of Formula (I) in Different Solvents

The crystal form A of the compound of formula (I) (30.00 mg, 44.08 µmοl, 1 *eq)* was separately suspended in ethanol (0.2 mL), isopropanol (0.2 mL), acetonitrile (0.2 mL), ethyl acetate (0.2 mL), tetrahydrofuran (0.2 mL), ethanol/water=20/1 (0.2 mL), isopropanol/water=20/1 (0.2 mL) and acetonitrile/water=20/1 (0.2 mL) at 20~30°C for 2 days, filtered under nitrogen protection and dried, and the test results of the crystal form are as shown in Table 8.

**Table 8 Stability test of the crystal form A of the compound of formula (I) in different solvents**

| **No.** | **Solvent** | **Appearance (2 days)** | **Result** |
|---|---|---|---|
| 1 | Ethanol | Suspension | Crystal form A |
| 2 | Isopropanol | Suspension | Crystal form A |
| 3 | Acetonitrile | Suspension | Crystal form A |
| 4 | Ethyl acetate | Suspension | Crystal form A |
| 5 | Tetrahydrofuran | Suspension | Crystal form A |
| 6 | Ethanol-water(20:1) | Suspension | Crystal form A |
| 7 | Isopropanol-water(20:1) | Suspension | Crystal form A |
| 8 | Acetonitrile-water(20:1) | Suspension | Crystal form A |

Conclusion: The crystal form A of the compound of formula (I) can be stable in a variety of solvents, with good reproducibility and easy to obtain.

### Embodiment 6: Exploration of Other Crystal Forms of the Compound of Formula (I)

Purpose: Explore obtaining other crystal forms by changing crystallization conditions.

**Table 9 Exploration of other crystal forms of the compound of formula (I)**

| **No.** | **Solvent** | **Conditions** | **Result** |
|---|---|---|---|
| 1 | THF:H₂O=3:1 | First dissolved at room temperature, cooled to 0~5°C, added with a crystal seed of crystal form A, and tried to crystallize | Some solid was precipitated, and all was dissolved when heated to room temperature, and the proportion of water was large |
| 2 | THF:H₂O=4:1 | First dissolved at room temperature, cooled down to 0~5°C, added with a crystal seed of crystal form A, and tried to crystallize | Some solid was precipitated, and all was dissolved when heated to room temperature, and the proportion of water was large |
| 3 | THF:H₂O=10:1 | First dissolved at 40°C, cooled to 0~5°C, and tried to crystallize | No crystal was obtained |
| 4 | EtOH:H₂O=10:1 | First dissolved and then cooled down | No crystal was obtained |
| 5 | EtOH:H₂O=20:1 | First dissolved and then cooled down | No crystal was obtained |
| 6 | IPA:H₂O=10:1 | First dissolved and then | No crystal was |
| | | cooled down | obtained |
| 7 | IPA:H₂O=20:1 | First dissolved and then cooled down | No crystal was obtained |
| 8 | acetone:H₂O=20:1 | First dissolved and then cooled down | No crystal was obtained |

Conclusion: The crystal form A of the compound of formula (I) was first dissolved in different aqueous systems and then crystallized, and no other crystal forms were obtained.

### Embodimet 7: Study on the Solid Stability of the crystal form A of the compound of formula (I)

The crystal form A of the compound of formula (I) was spread to a thin layer and placed in complete exposure to conditions of high temperature, room temperature and high humidity, light, and high temperature and high humidity, and samples were taken according to prescribed time points to test XRPD, appearance, content and related substances, compared with the results of the 0 day sample.

**Table10: Solid stability study results of the crystal form A of the compound of formula (I)**

| **Study conditions of the sample** | **XRPD result** | **Total impurities %** | **Purity %** | **Appearance** |
|---|---|---|---|---|
| 0 day | Crystal form A | 14.4 | 85.6 | Almost white powder |
| 60°C-5 days (open) | Crystal form A | 12.7 | 87.3 | Almost white powder |
| 60°C-10 days (open) | Crystal form A | 13.4 | 86.6 | Almost white powder |
| 25°C/75% RH-5 days (open) | Crystal form A | 12.6 | 87.4 | Almost white powder |
| 25°C/75% RH-10 days (open) | Crystal form A | 14.6 | 85.4 | Almost white powder |
| Light -10 days (visible light intensity 5000 lux and ultraviolet intensity 90µw/cm², open) | Crystal form A | 13.6 | 86.4 | Almost white powder |
| Light control -10 days | Crystal form A | 13.2 | 86.8 | Almost white powder |

Conclusion: The crystal form A of the compound of formula (I) was stable under high temperature, high humidity and light conditions, and the related substances and contents did not change significantly at 60°C, 25°C/75% RH and in light conditions.

### Evaluation of Biological Activity of the Crystal Form A of the Compound of formula (I)

### Experimental Example 1: Detection of Antibacterial Activity (MIC) of the Crystal Form A of the Compound of Formula (I)

Three strains of *Klebsiella pneumonia,* ATCC BAA-205 (TEM-1/SHV-1/SHV-12), ATCC BAA-1705 (KPC-2), ATCC BAA-2470 (NDM-1), *Enterobacter cloacae* ATCC BAA-1143 (AmpC), two strains of *Escherichia coli* ATCC BAA-2523(OXA-48), ATCC 25922 were used to determine the minimum inhibitory concentration (MIC) of each of the compounds by a micro-liquid dilution method according to the Institute of Clinical and Laboratory Standard (CLSI) requirements. A 2-fold serial dilution of the compound (final concentration ranging from 0.125 µg/ml to 128 g/ml) was added to a round-bottom 96-well plate (Catalog #3788, Corning), fresh monoclonal bacteria were selected from an overnight cultured Mueller Hinton II Agar medium plate (MHA, Cat. No. 211438, BD BBL^{™}) and suspended in sterile saline, the concentration was adjusted to 1×10⁸ CFU/mL, and then the solution was diluted by using a cation-adjusted Hinton Mueller culture medium Cation-Adjusted Mueller Hinton II Broth (MHB, Catalog #212332, BD BBL^{™}) to a concentration of 5×10⁵ CFU/ml, and 100 µl of the solution was added to a round bottom 96-well plate containing the drugs. The plate was placed upside down at 37° C and the MIC value was read after 20-24 h of incubation, and the lowest drug concentration that inhibited the growth of bacteria was set as MIC. For the results see Table 1. Colony-Forming Units (CFU) refers to the total number of bacterial colonies per unit volume.

**Table 11 Detection of antibacterial effect MIC (µg/mL) data of the crystal form A of the compound of formula (I)**

| **Compound** | **Bacterial strain** | ***K. pneumoniae*** | | | ***E. cloacae*** | ***E. coli*** | ***E. coli*** |
|---|---|---|---|---|---|---|---|
| | | **ATCC BAA-205** | **ATCC BAA-1705** | **ATCC BAA-2470** | **ATCC BAA-1143** | **ATCC BAA-2523** | **ATCC 25922** |
| | **Drug resistance gene** | **Class A (TEM-1**/**SHV-1/SHV-12)** | **Class A (KPC-2)** | **Class B (NDM-1)** | **Class C (AmpC)** | **Class D (OXA-48)** | **-** |
| Crystal form A of the compound of formula (I) | | 0.5 | 1 | 0.25 | 0.5 | 0.125 | 0.25 |

Conclusion: The crystal form A of the compound of formula (I) has a good inhibitory effect on a variety of bacteria.

### Experimental Example 2: Pharmacokinetics Study of the Crystal Form A of the Compound of Formula (I) in Rats in Vivo

The pharmacokinetic characteristics in rodents after intravenous administration of the compound was tested with standard scheme. Specifically, SD male rats at the age of 7-10 weeks were administered intravenously with the candidate compound in the experiment. The compound was dissolved in salt water to prepare a clear solution. The plasma samples are collected and analyzed by LC-MS/MS, and the pharmacokinetic parameters were calculated. For the pharmacokinetic parameters of the crystal form A of the compound of formula (I) see Table 12.

**Table 12 Pharmacokinetic data of the crystal form A of the compound of formula (I) in rats in vivo**

| **Pharmacokinetic parameters** | **Injection at 100 mg / kg Dissolved in saline** | **Injection at 300 mg / kg Dissolved in saline** |
|---|---|---|
| C₀ (nmol/L) Initial concentration | 581401 | 1375717 |
| Cl (mL/min/kg) | 17.2 | 18.4 |
| Clearance rate | | |
| V_{dss} (L/kg) | 0.378 | 0.463 |
| Apparent volume of distribution | | |
| T_{1/2}(h) | 0.311 | 0.362 |
| Half-life period | | |
| AUC₀₋ₗₐₛₜ (nM.hr) | 166093 | 467222 |
| Area under plasma concentration-time curve | | |

Conclusion: The exposure of the compound of the present disclosure increases linearly, which can give full play to the advantage of the maximum dosage of the drug within the safety range in the later research to obtain better clinical effect, with good drug manufacture prospects.

### Experimental Example 3: Spontaneous drug resistance mutation rate (FOR) of the crystal form A of the compound of formula (I) on Escherichia coli ATCC 25922

*Escherichia coli* ATCC 25922 was used to carry out a micro-liquid dilution method according to the Institute of Clinical and Laboratory Standard (CLSI) requirements, the colony for selection with a total colony count of 2.60E+08CFU in Colony-Forming Units (CFU) was selected, and incubated for 24 h and 48 h at 2, 4, 8, 16, 32-fold of minimum inhibitory concentration (MIC), and the drug resistance frequency was calculated. See Table 13 for details.

**Table 13 Spontaneous drug resistance mutation rate of the crystal form A of the compound of formula (I)**

| **Test substance** | **Selected total colony count (CFU)** | **Selection criteria** | **Drug resistance frequency (24 h)** | **Drug resistance frequency (48 h)** | **Drug resistance frequency (72 h)** |
|---|---|---|---|---|---|
| Crystal form A of the compound of formula (I) | 2.60E+08 | 2×MIC | <3.85E-09 | <3.85E-09 | =3.85E-09 |
| | | 4× MIC | <3.85E-09 | <3.85E-09 | <3.85E-09 |
| | | 8× MIC | <3.85E-09 | <3.85E-09 | <3.85E-09 |
| | | 16× MIC | <3.85E-09 | <3.85E-09 | <3.85E-09 |
| | | 32× MIC | <3.85E-09 | <3.85E-09 | <3.85E-09 |

Conclusion: Compound (I) has a lower drug resistance rate with 2-fold of MIC, and has a better prospect for clinical application.

The crystal form A of the compound (I) provided by the present disclosure has a stable nature, a good solubility, and a good hygroscopy, with good drug manufacture prospects.

The crystal form A of the compound (I) provided by the present disclosure is easy to obtain, and the reagents used are common, easy to obtain in the market and at low prices; the stable single crystal form can be obtained by various preparation methods, with simple operation and mild conditions, and with good purity and high yield.

### Example 4: Drug Efficacy of the Crystal Form A of the compound of formula (I) in thigh muscle infection model in vivo

78 CD-1 female mice were divided into 26 cages, 3 mice per cage. The day of infection is counted as the 0 day.

150 mg/kg immunosuppressant cyclophosphamide was intraperitoneally injected on Day-4, and 100 mg/kg immunosuppressant cyclophosphamide was intraperitoneally injected again on Day-1 to obtain immunodeficient mice.

The strain *Klebsiella pneumoniae* ATCC-BAA 2470 (NDM+) was recovered on an MHA plate on Day-1. The recovered colonies were picked and dissolved in sterile saline to prepare a bacterial solution with a concentration of 5.95E+07 CFU/mL for use in mouse thigh muscle infection. The infection starting time was counted as 0h, and 100µL of bacterial solution is injected into the thigh muscle of each mouse at 0h, that is, the inoculation amount is 5.95E+06 CFU/mouse. 2h after infection, the drugs were administered according to the experimental scheme. The specific experimental scheme is as follows (see Table 14):
(1) 2h after infection: At the end point of mice of the 25th cage, the thigh muscle tissue was taken and placed in 10 mL sterile saline, the tissue was homogenized by a homogenizer, and the homogenate was dotted on a plate with gradient dilution, two duplications for each mouse.
(2) 2h after infection: Mice in the 1st to 24th cage were subcutaneously administered separately with the crystal form A of the compound of foluma (I) at a volume of 10 mL/kg according to the mouse body weight for treatment. The mice in the 1st to 4th cage were administered at a total daily dose of 1600 mg/kg, the mice in the 5th to 8th cage were administered at a total daily dose of 480 mg/kg, the mice in the 9th to 12th cage were administered at a total daily dose of 160 mg/kg, the mice in the 13th to 16th cage were administered at a total daily dose of 48 mg/kg, the mice in the 17th to 20th cage were administered at a total daily dose of 16 mg/kg, the mice in the 21th to 24th cage were administered at a total daily dose of 4.8 mg/kg, and each dosage group was administered separately in four different dosage dividing manners, which were q3h, q6h, q12h and q24h separately.
(3) 26 h after infection: The endpoint of the experiment was at 26 h after infection, the thigh muscle tissue of the mice of the 1st to 26th cage was taken and placed in 10 mL sterile saline, the tissue was homogenized by a homogenizer, and the homogenate was dotted on a plate with gradient dilution, two duplications for each mouse. The bacterial load of the thigh muscle tissue of the mice was counted, and for the experimental results see Table 15.

**Table 14 Experimental scheme**

| **Experimental group** | **Number of animals** | **Total daily dose of the crystal form A of the compound of formula (I) mg/kg** | **Single administration dose mg/kg** | **Administration frequency s.c.** | **Experiment end point** | **Testing index** |
|---|---|---|---|---|---|---|
| 1 | 3 | 1600 | 60 | q3h | 26h | The bacterial load of the thigh muscle tissue |
| **2** | 3 | | 400 | q6h | 26h | |
| **3** | 3 | | 800 | q12h | 26h | |
| **4** | 3 | | 1600 | q24h | 26h | |
| **5** | 3 | 480 | 60 | q3h | 26h | |
| **6** | 3 | | 120 | q6h | 26h | |
| 7 | 3 | | 240 | q12h | 26h | |
| **8** | 3 | | 480 | q24h | 26h | |
| **9** | 3 | 160 | 20 | q3h | 26h | |
| **10** | 3 | | 40 | q6h | 26h | |
| **11** | 3 | | 80 | q12h | 26h | |
| **12** | 3 | | 160 | q24h | 26h | |
| **13** | 3 | 48 | 6 | q3h | 26h | |
| **14** | 3 | | 12 | q6h | 26h | |
| **15** | 3 | | 24 | q12h | 26h | |
| **16** | 3 | | 48 | q24h | 26h | |
| **17** | 3 | 16 | 2 | q3h | 26h | |
| **18** | 3 | | 4 | q6h | 26h | |
| **19** | 3 | | 8 | q12h | 26h | |
| **20** | 3 | | 16 | q24h | 26h | |
| **21** | 3 | 4.8 | 0.6 | q3h | 26h | |
| **22** | 3 | | 1.2 | q6h | 26h | |
| **23** | 3 | | 2.4 | q12h | 26h | |
| **24** | 3 | | 4.8 | q24h | 26h | |
| **25** | 3 | 2h control groups | ---- | ---- | 2h | |
| **26** | 3 | 26h infected groups | ---- | ---- | 26h | |

**Table 15 Experimental results of the bacterial load of mouse thigh muscle tissue at the end point 26h**

| **Experimental group** | **Number of animals** | **Total daily dose of the crystal form A of the compound of formula (I) mg/kg** | **Single administration dose mg/kg** | **Administration frequency s.c.** | **Average CFU of Mouse (CFU/ Mouse)** |
|---|---|---|---|---|---|
| **1** | 3 | 1600 | 60 | q3h | 2.18E+06 |
| **2** | 3 | | 400 | q6h | 2.45E+06 |
| **3** | 3 | | 800 | q12h | 3.80E+08 |
| **4** | 3 | | 1600 | q24h | 9.02E+09 |
| **5** | 3 | 480 | 60 | q3h | 2.20E+06 |
| **6** | 3 | | 120 | q6h | 2.18E+08 |
| 7 | 3 | | 240 | q12h | 2.88E+09 |
| **8** | 3 | | 480 | q24h | 8.73E+09 |
| **9** | 3 | 160 | 20 | q3h | 2.10E+06 |
| **10** | 3 | | 40 | q6h | 4.67E+08 |
| **11** | 3 | | 80 | q12h | 7.88E+09 |
| **12** | 3 | | 160 | q24h | 1.22E+10 |
| **13** | 3 | 48 | 6 | q3h | 4.07E+06 |
| **14** | 3 | | 12 | q6h | 1.57E+09 |
| **15** | 3 | | 24 | q12h | 8.70E+09 |
| **16** | 3 | | 48 | q24h | 1.32E+10 |
| **17** | 3 | 16 | 2 | q3h | 7.77E+07 |
| **18** | 3 | | 4 | q6h | 2.92E+09 |
| **19** | 3 | | 8 | q12h | 6.93E+09 |
| **20** | 3 | | 16 | q24h | 1.44E+10 |
| **21** | 3 | 4.8 | 0.6 | q3h | 7.22E+08 |
| **22** | 3 | | 1.2 | q6h | 4.55E+09 |
| **23** | 3 | | 2.4 | q12h | 7.60E+09 |
| **24** | 3 | | 4.8 | q24h | 1.27E+10 |
| **25** | 3 | 2h control group | ---- | ---- | 2.95E+07 |
| **26** | 3 | 26h infected | ---- | ---- | 3.22E+10 |
| | | groups | | | |

Conclusion: The crystal form A of the compound of formula (I) of the present disclosure has significant antibacterial effect in vivo, and the dose-effect relationship is significant. Under the same daily dose, shortening the administration interval can significantly improve the efficacy in vivo.

## Claims

1. A pharmaceutically acceptable salt of a compound represented by formula (I), and a crystal form thereof,

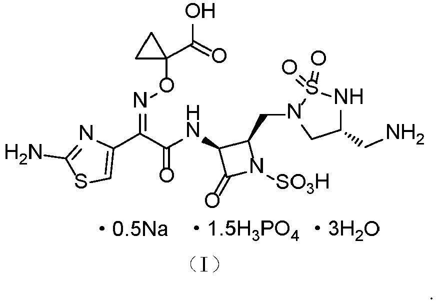

2. A crystal form A of a pharmaceutically acceptable salt of a compound represented by formula (I), 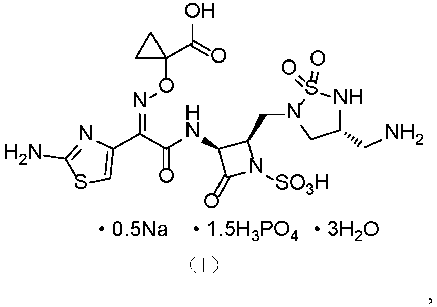 wherein the X-ray powder diffraction pattern thereof has characteristic diffraction peaks at the following 2θ angles: 15.05±0.20°, 19.11±0.20°, and 21.15±0.20°.

3. The crystal form A according to claim 2, wherein the X-ray powder diffraction pattern thereof has characteristic diffraction peaks at the following 2θ angles: 15.05±0.20°, 19.11±0.20°, 20.41±0.20°, 21.15±0.20°, 22.62±0.20°, 23.48±0.20°, 27.32±0.20°, and 29.25±0.20°.

4. The crystal form A according to claim 2, wherein the X-ray powder diffraction pattern thereof has characteristic diffraction peaks at the following 2θ angles: 9.10±0.20°, 11.29±0.20°, 12.76±0.20°, 15.05±0.20°, 16.39±0.20°, 19.11±0.20°, 20.41±0.20°, 21.15±0.20°, 22.62±0.20°, and 23.68±0.20°.

5. The crystal form A according to claim 2, wherein the X-ray powder diffraction pattern thereof has characteristic diffraction peaks at the following 2θ angles: 6.90±0.20°, 9.10±0.20°, 11.29±0.20°, 11.83±0.20°, 12.76±0.20°, 13.73±0.20°, 14.57±0.20°, 15.05±0.20°, 16.10±0.20°, 16.39±0.20°, 17.81±0.20°, 18.25±0.20°, 19.11±0.20°, 20.41±0.20°, and 21.15±0.20°.

6. The crystal form A according to claim 2, wherein the X-ray powder diffraction pattern thereof has characteristic diffraction peaks at the following 2θ angles: 6.90±0.20°, 9.10±0.20°, 11.29±0.20°, 11.83±0.20°, 12.76±0.20°, 13.73±0.20°, 15.05±0.20°, 16.39±0.20°, 17.81±0.20°, 19.11±0.20°, 20.41±0.20°, 21.15±0.20°, 21.64±0.20°, 22.62±0.20°, and 23.48±0.20°.

7. The crystal form A according to claim 2, wherein the X-ray powder diffraction pattern thereof has characteristic diffraction peaks at the following 2θ angles: 6.90±0.20°, 9.10±0.20°, 11.29±0.20°, 11.83±0.20°, 12.76±0.20°, 13.73±0.20°, 15.05±0.20°, 16.39±0.20°, 17.81±0.20°, 19.11±0.20°, 20.41±0.20°, 21.15±0.20°, 21.64±0.20°, 22.62±0.20°, 23.48±0.20°, 25.73±0.20°, 26.43±0.20°, 27.32±0.20°, 28.42±0.20°, and 29.25±0.20°.

8. The crystal form A according to claim 2, wherein the X-ray powder diffraction pattern thereof has characteristic diffraction peaks at the following 2θ angles: 6.904°, 9.095°, 11.285°, 11.833°, 12.757°, 13.732°, 14.573°, 15.050°, 16.100°, 16.389°, 17.807°, 18.251°, 19.113°, 20.413°, 20.790°, 21.147°, 21.641°, 22.615°, 23.484°, 23.676°, 24.002°, 24.812°, 25.728°, 26.433°, 26.655°, 27.323°, 27.636°, 28.420°, 29.248°, 29.922°, 30.358°, 30.733°, 31.507°, 31.830°, 32.049°, 32.509°, 32.843°, 33.130°, 33.837°, 34.346°, 34.895°, 35.500°, 36.037°, 36.467°, 37.098°, 37.573°, 37.875°, and 38.797°.

9. The crystal form A according to any one of claims 2-8, wherein the thermogravimetric analysis curve thereof has a weight loss of 6.657% at 140.000°C ±3.000°C and a weight loss of 12.576% at 205.000°C±3.000°C.

10. The crystal form A according to any one of claims 2-8, wherein the thermogravimetric analysis curve thereof is basically as shown in FIG. 2.

11. The crystal form A according to any one of claims 2-8, wherein the differential scanning calorimetric curve thereof has an endothermic peak at 100.28°C±3.00°C, an endothermic peak at 127.61°C±3.00°C, an exothermic peak at 200.70°C±3.00°C, and an endothermic peak at 266.36°C±3.00°C.

12. The crystal form A according to any one of claims 2-8, wherein the differential scanning calorimetric curve thereof is basically as shown in FIG. 3.

13. A crystal form A of a pharmaceutically acceptable salt of a compound represented by formula (I), wherein the XRPD pattern thereof is basically as shown in FIG. 1,

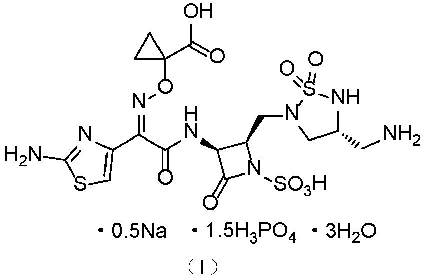

14. The crystal form A according to claim 13, wherein the thermogravimetric analysis curve thereof has a weight loss of 6.657% at 140.000°C ±3.000°C and a weight loss of 12.576% at 205.000°C±3.000°C.

15. The crystal form A according to claim 13, wherein the thermogravimetric analysis curve thereof is basically as shown in FIG. 2.

16. The crystal form A according to claim 13, wherein the differential scanning calorimetric curve thereof has an endothermic peak at 100.28°C±3.00°C, an endothermic peak at 127.61°C±3.00°C, an exothermic peak at 200.70°C±3.00°C, and an endothermic peak at 266.36°C±3.00°C.

17. The crystal form A according to claim 13, wherein the differential scanning calorimetric curve thereof is basically as shown in FIG. 3.

18. An application of the pharmaceutically acceptable salt and the crystal form thereof according to claim 1 in preparation of medicaments for treating bacterial infection-related diseases.

19. An application of the crystal form A according to any one of claims 2~17 in preparation of medicaments for treating bacterial infection-related diseases.
